(19) Europäisches Patentamt
European Patent Office
Office européen des brevets

(11) EP 2 732 052 B1

(12) **EUROPEAN PATENT SPECIFICATION**

(45) Date of publication and mention of the grant of the patent:
**16.11.2016 Bulletin 2016/46**

(21) Application number: **12761808.0**

(22) Date of filing: **13.07.2012**

(51) Int Cl.:
***C12Q 1/68*** *(2006.01)*

(86) International application number:
**PCT/IB2012/001702**

(87) International publication number:
**WO 2013/011378 (24.01.2013 Gazette 2013/04)**

(54) **DIAGNOSTIC MICRORNA PROFILING IN CUTANEOUS T-CELL LYMPHOMA (CTCL)**

DIAGNOSTISCHE MICRORNA-PROFILIERUNG BEI KUTANEM T-ZELL-LYMPHOM (CTCL)

PROFILAGE DIAGNOSTIQUE DE MICROARN DANS LE LYMPHOME CUTANÉ À CELLULES T (CTCL)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.07.2011 US 201161508231 P**
**18.07.2011 US 201161508838 P**

(43) Date of publication of application:
**21.05.2014 Bulletin 2014/21**

(73) Proprietors:
- **Leo Pharma A/S**
  **2750 Ballerup (DK)**
- **Copenhagen University**
  **1165 Copenhagen K (DK)**
- **Rigshospitalet**
  **2100 Copenhagen Ø (DK)**
- **Gentofte Hospital**
  **2900 Hellerup (DK)**
- **Exiqon A/S**
  **2950 Vedbaek (DK)**

(72) Inventors:
- **RALFKIAER, Ulrik**
  **2100 København Ø (DK)**
- **HAGEDORN, Peter**
  **2970 Hørsholm (DK)**
- **AHLER, Charlotte, Busch**
  **2630 Tåstrup (DK)**
- **GEISLER, Carsten**
  **1266 København K (DK)**
- **WOETMANN, Anders**
  **2200 København (DK)**
- **SKOV, Lone**
  **2950 Vedbaek (DK)**
- **ØDUM, Niels, Feentved**
  **1306 København K (DK)**
- **RALFKIER, Elizabeth**
  **2970 Hørsholm (DK)**

(74) Representative: **Høiberg A/S**
**Adelgade 12**
**1304 Copenhagen K (DK)**

(56) References cited:
- **MARLOES S VAN KESTER ET AL: "miRNA expression profiling of mycosis fungoides", MOLECULAR ONCOLOGY, ELSEVIER, AMSTERDAM, NL, vol. 5, no. 3, 16 February 2011 (2011-02-16), pages 273-280, XP028221235, ISSN: 1574-7891, DOI: 10.1016/J.MOLONC.2011.02.003 [retrieved on 2011-02-24] cited in the application**
- **BALLABIO E ET AL: "MicroRNA expression in Sezary syndrome: identification, function, and diagnostic potential", BLOOD, AMERICAN SOCIETY OF HEMATOLOGY, US, vol. 116, no. 7, 19 August 2010 (2010-08-19), pages 1105-1113, XP002612685, ISSN: 0006-4971, DOI: 10.1182/BLOOD-2009-12-256719 [retrieved on 2010-05-06] cited in the application**
- **LINE MARIE HOLST ET AL: "Reproducible pattern of microRNA in normal human skin", EXPERIMENTAL DERMATOLOGY, vol. 19, no. 8, 1 August 2010 (2010-08-01) , pages e201-e205, XP55043455, ISSN: 0906-6705, DOI: 10.1111/j.1600-0625.2009.01049.x cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **M G NARDUCCI ET AL: "MicroRNA profiling reveals that miR-21, miR486 and miR-214 are upregulated and involved in cell survival in Sézary syndrome", CELL DEATH AND DISEASE, vol. 2, no. 4, 1 April 2011 (2011-04-01), page e151, XP55043454, DOI: 10.1038/cddis.2011.32 cited in the application**
- **U. RALFKIAER ET AL: "Diagnostic microRNA profiling in cutaneous T-cell lymphoma (CTCL)", BLOOD, vol. 118, no. 22, 24 August 2011 (2011-08-24), pages 5891-5900, XP55043495, ISSN: 0006-4971, DOI: 10.1182/blood-2011-06-358382**
- **MARCHINA F. BENNER ET AL: "Primary cutaneous anaplastic large cell lymphoma shows a distinct miRNA expression profile and reveals differences from tumor-stage mycosis fungoides", EXPERIMENTAL DERMATOLOGY, vol. 21, no. 8, 10 July 2012 (2012-07-10), pages 632-634, XP55043497, ISSN: 0906-6705, DOI: 10.1111/j.1600-0625.2012.01548.x**

## Description

### FIELD OF THE INVENTION:

The present invention relates to the field of cancer-diagnostics. In particular the invention relates to a microRNA expression signature that allows discriminating non malignant (inflammantory) skin samples from skin samples of cutaneous T-cell lymphomas (CTCL).

### BACKGROUND OF THE INVENTION:

Cutaneous T-cell lymphomas (CTCL) are the most frequent primary lymphomas of the skin, with mycosis fungoides (MF) being the most prevalent clinical form accounting for around 60% of new cases (Trautinger, 2006).

In early disease stages, which can last several years, MF presents as flat erythematous skin patches resembling inflammatory diseases such as dermatitis or psoriasis. In later stages, MF lesions gradually form plaques and overt tumors and may disseminate to lymph nodes and internal organs. The early skin lesions of this disease contain numerous inflammatory cells, including a large quantity ofT cells with a normal phenotype as well as a small population of T cells with a malignant phenotype.

The infiltrate primarily consists of non-malignant T helper 1 (Th1) cells, regulatory T cells (Treg), and cytotoxic $CD8^+$ T cells, which to some degree seem to control the malignant T cells (Lee, 1999; Gjerdrum, 2007). The malignant T cells typically exhibit the phenotype of mature $CD4^+$ memory T cells and are normally of clonal origin (Rosen, 2006). T cells with a malignant phenotype are characterized by epidermotropism and are preferentially present in the upper parts of the skin, whereas T cells with a normal phenotype primarily are detected in the lower portions of the dermis. The epidermal T cells are sometimes found in patterns of Pautrier micro-abscesses, which are collections of T cells adherent to dendritic processes of Langerhans cells. During disease development, the epidermotropism is gradually lost concomitant with an increase in malignant, and a decrease in non-malignant, infiltrating T cells.

The etiology of CTCL remains poorly understood, and occupational exposures, infectious agents, and genetic mutations have been proposed as etiological factors, but no evidence of causation has been provided (Dereure, 2002). Instead, an aberrant expression and function of transcription factors and regulators of signal transduction is a characteristic feature of CTCL. Accordingly, it has been hypothesized that a dysfunctional regulation of signal molecules and cytokines plays a key role in the malignant transformation and epigenetic modifications such as aberrant gene methylation and histone de-acetylation are clearly involved in the pathogenesis of CTCL (Doorn, 2009;Girardi, 2004).

Early diagnosis is difficult because of the great clinical and histological resemblance to benign inflammatory diseases such as dermatitis or psoriasis. A definitive diagnosis from a skin biopsy requires the presence of convoluted lymphocytes, a band-like upper dermal infiltrate, and epidermal infiltrations with Pautrier abscesses, but all of these features are often not present in early stages and the histological picture is often difficult to interpret. Histological examinations can be supplemented by surrogate markers such as low CD7 and intermediate CD4 expression and T cell-receptor clonality, none of which are specific for CTCL. Moreover, these methods are laborious and no definitive disease markers exist. Accordingly, a definitive diagnosis may require the review of multiple biopsies over an extended period of time by an experienced pathologist. Thus, patients are often left in uncertainty for an extended period of time and subjected to different kinds of inefficient treatments.

MicroRNAs (miRNAs or miRs) are an abundant class of short endogenous RNAs that act as posttranscriptional regulators of gene expression by base-pairing with their target mRNAs.Specifically, miRNAs prevent mRNA translation and/or mediate target mRNA degradation. miRNAs are 19-25 nucleotide (nt) RNAs that are processed from longer endogenous hairpin transcripts (Ambros et al. 2003, RNA 9: 277-279). To date more than 6000 miRNAs have been identified in humans, worms, fruit flies and plants according to the miRNA registry database release 11.0 in April 2008, hosted by Sanger Institute, UK.

Recent data indicate that several miRNAs, are differentially expressed and possibly also involved in the pathogenesis of cancer (Garzon, 2009)[12]. Thus, miR-21 expression is up-regulated and appears to play a role in the regulation of apoptosis in malignant T cells obtained from patients with Sezary Syndrome (SS), a leukemic variant of CTCL. These findings are in keeping with studies in other cancers where miRNAs have been ascribed a key role in cancer development and metastasis. Indeed, specific miRNAs are directly involved in the malignant transformation as they can function as oncogenes and tumor suppressors (Krejsgaard, 2009).

Early diagnosis of CTCL has important consequences concerning therapeutic options and determination of prognosis. Unfortunately, early diagnosis of CTCL has proven difficult because of the great clinical, pathological, and histological resemblance to benign inflammatory skin diseases Accordingly, there is a need for an early and precise malignancy diagnosis which would allowing targeted treatment with established treatment modalities such as topical chemotherapy or phototherapy.

## SUMMARY OF THE INVENTION:

The present invention provides a method based on a diagnostic miRNA classifier which allow a fast and accurate classification of skin specimens as being malignant or benign. Accordingly, the invention pertains to a method for classifying a test skin cell sample from an individual with an inflammatory skin disease as cutaneous lymphomas, as defined in claim 1, comprising:

detecting microRNA expression levels at least of one of miR-155 and miR-326, and both miR-203 and miR-205,calculating a clinical score (S) of the test cell sample based on a dataset comprising the expression levels of said microRNAs, and classifying the test cell sample as cutaneous lymphomas or not based on the value of the clinical score.

## DEFINITIONS

Prior to a discussion of the detailed embodiments of the invention is provided a definition of specific terms related to the main aspects and embodiments of the invention.

The terms "cutaneous lymphoma", "cutaneous T-cell lymphoma, "mycosis fungoides" and "Sezary syndrome" are used and in accordance with the WHO-EORTCguidelines as described(Olsen, 2007; Willemze, 2005; Foss, 2011; Burg, 2005).

The terms "miR", "miRNA" and "microRNA" are used synonymously and refer to a class of about 18-25 nucleotides (nt) long non-coding RNAs derived from endogenous genes. They are processed from longer (ca 75 nt) hairpin-like precursors termed pre-miRs. MicroRNAs assemble in complexes termed miRNPs and recognize their targets by antisense complementarity. If the microRNAs match 100% their target, i.e. the complementarity is complete, the target mRNA is cleaved, and the miR acts like a siRNA. If the match is incomplete, i.e. the complementarity is partial, then the translation of the target mRNA is blocked.

As used herein the terms "let-7b", "miR-103", "miR-155", "miR-184", "miR-191", "miR-203", "miR-205", "miR-24", "miR-299-5p", "miR-326", "miR-34b", "miR-423-5p", "miR-663b", "miR-711" or "miR-718" refer to the human miR sequences found in miR registry database release 12.0 or later and hosted by Sanger Institute, UKas well as their animal equivalents. Except for miR-711 and miR-718, all miRs are human miR sequences commonly referred to by the prefix "hsa-", e.g. hsa-miR-155 refer to the human miR-155. miR-711 and miR-718 were not published until registry database release 14.0.

As used herein the term "detecting the level of a miR" refer to the quantification of said miR. One way of quantification is described in the Examples i.e. qRT-PCR. However, the miR may be quantified in a multitude of other ways e.g. by arrays, northern blots, dot blots, RN'ase protection assays, quantitative mass spectroscopy or various quantitative PCR-based methods such as the TaqMan assay or the UniRT assay used in the examples.

The term "expression", as used herein, refers to the transcription and/or accumulation of RNA-molecules within a call.

In the present context the terms "level of expression of a miR" and "level of a miR" are used synonymously as a measure of the "amount of a specific miR" that is detected in the sample. The "amount of a specific miR" may be expressed in either absolute or relative measures and refers to values obtained by both quantitative, as well as qualitative methods. One particularly preferred measure of the "amount of a specific miR" is the Crossing point (Cp) value obtained by real-time qRT-PCR as described in the examples. Another preferred measure of the "amount of a specific miR" is the "threshold cycle value (Ct)" value likewise obtained by real-time qRT-PC.R as described in the examples. The Cp and the Ct measures of the "amount of a specific miR" provide roughly similar measures, see Bustin, S.A. (ed.) A-Z of quantitative PCR, IUL Biotechnology Series 5 (2004) 882 pages. Whether to choose Cp or Ct is largely a matter of choice of the machine the assay tied to andperformed on. If the amplification is performed in a LightCycler® 480 Real-Time-PCR System using the Roche LC software the amount of a specific miR is expressed by the Cp. If the amplification is performed in Applied Biosystems ABI Prism 7900HT 384-well instrument using the software provided with it the amount of a specific miR is expressed by the Ct.

The term "level" designates relative as well as absolute amounts of the miRs referred to.

The terms "Q-PCR" or "q-PCR" refers to quantitative polymerase chain reaction. Q-PCR is highly sensitive method for quantifying the amounts of specific DNA (and RNA) species in a test sample. As quantification of RNA by the PCR technique requires that the RNA is reverse transcribed it is often referred to as "qRT-PCR" or "RT-Q-PCR" to indicate that quantitative PCR is used to quantify specific RNAs. A thorough treatise of the Q-PCR and qRT-PCRtechniques can be found in Bustin, S.A. (ed.) A-Z of quantitative PCR, IUL Biotechnology Series 5 (2004) 882 pages.

" UniRT" is a novel Q-PCR method. The method is described in Example 4 and International Patent Application WO 2010/085966.

**DETAILED DISCLOSURE OF THE INVENTION:**

In the present study we used microarrays for an initial screening of miRNAs with a potential ability to distinguish between malignant (CTCL) and benign inflammatory skin disorders such as psoriasis, atopic dermatitis, and contact dermatitis. Five miRNAs (miR-203, miR-205, miR-326, miR-663b, and miR-711) were identified, which discriminated with high accuracy (>90%) between malignant and benign conditions in a total of 198 patients including an initial training sef of 90 patients, a test set of 58 patients and an independent cohort of 50 patients, example 1, fig 1. Importantly, the expression pattern of four out of five miRNAs (miR-203, miR-205, miR-326 and miR-663b) was verified using qRT-PCR on RNA samples from 103 patients. Accordingly one embodiment of the. present invention is a method for classifying a test skin cell sample from an individual with an inflammatory skin disease as cutaneous lymphomas comprising detecting microRNA (miR) expression levels of a selection of miRs comprising e.g. miR-326, miR-203, miR-205 and miR-663b in the test skin cell sample, calculating a clinical score (S) of the test cell sample based on a dataset comprising the expression levels of said microRNAs, and classifying the test cell sample as cutaneous lymphomas or not based on the value of the clinical score.

Recent studies on subpopulations of CTCL patients identified miR-155 as differentially expressed in Sezary Syndrome (SS) patients and advanced (tumor-stage) mycosis fungoides (MF) patients, respectively (Fits, 2011; Kester, 2011). Interestingly, we were only able to verify the miR-155 expression data by the qRT-PCR technique. However, with respect to miR-155 we were able to confirm and extend the findings of van Kester et al. Furthermore, our qRT-PCR results showed that miR-155 was one of the most significantly differentially expressed micorRNAs in CTCL.

Therefore, one embodiment of the present invention is a method wherein the levels of microRNA miR-155, miR-326, miR-203, miR-205 and miR-663b are detected and used to calculate a clinical score (S) of the test cell sample.

Using the nearest shrunken centroid algorithm on qRT-PCR data, miR-155, miR-203, and miR-205were identified as the most discriminative set of miRNAs, see example 3. Accordingly, in one preferred embodiment of the present invention, the levels of miR-155 and both miR-203 and miR-205 are detected and used to calculate a clinical score (S) of the test cell sample.

Example 3, fig 3B, show that the differential expression was also clearly confirmed for miR-203,miR-205, andmiR-326 with P-values below $10^{-11}$.Thus in one preferred embodiment of the present invention, the levels of microRNA miR-326 and both miR-203 and miR-205 are detected and used to calculate a clinical score (S) of the test cell sample.

As miR-155 and miR-326 are the 2 most upregulated miRs in cutaneous lymphoma biopsies a further preferred embodiment of the present invention is an method comprising detecting the levels of miR-326 and both miR-203 and miR-205 and used this data to calculate a clinical score (S) of the test cell sample.

In a still further embodiment of the present method the levels of microRNA miR-155, miR-326, miR-203 and miR-205 are detected and used to calculate a clinical score (S) of the test cell sample.

Cutaneous T-cell lymphomas (CTCL) are the most frequent primary lymphomas of the skin, therefore a further preferred embodiment of the present invention is a method wherein the method is used to differentiate benign test skin samples and test skin samples wherein the cutaneous lymphoma is cutaneous T-cell Lymphoma (CTCL).

According to the invention, the clinical score (S) may be calculated in a number of different ways. Importantly, when the score is calculated as the difference between the crossing point-value (Cp) determined by qRT-PCR of miR-155 and the average Cp of miR-203 and miR-205 the.score was found to distinguish patients with CTCL from benign skin diseases with very high sensitivity, specificity, and classification accuracy (95%), see example 3, fig. 7. Thus, in one embodiment, the clinical score is calculated as a ratio of the expression level of miR-155 and the average expression levels of miR-203 and miR-205.

As seen in example 3 (fig 3B) differential expression was clearly confirmed for miR-203, miR-205, and miR-326 with P-values below $10^{-13}$. Accordingly, in another embodiment, the clinical score is calculated as a ratio of the expression level of miR-326 and the average expression levels of miR-203 and miR-205.

In an even further embodiment the clinical score is calculated as a ratio of the expression level of miR-155 and miR-326 relative to the average expression levels of miR-203 and miR-205.

MiR's may be quantified in a number of ways e.g. by arrays, northern blots, dot blots, RN'ase protection assays, quantitative mass spectroscopy or various quantitative PCR-based techniques.

The enzyme used in the PCR-technique is in most instances a temperature resistant DNA polymerase, thus in order to provide the necessary DNA template for the DNA polymerase to act on, RNA's are copied into their DNA complement by the action of a reverse transcriptase, before subjected to PCR. In the subsequent steps of the method the DNA-copies (often referred to as cDNA) is subjected to a quantitative PCR (q-PCR). The collective method of reverse transcribing the RNA in a sample an subsequently quantifying it by q-PCR is referred to quantitative reverse transcription polymerase chain reaction, or "qRT-PCR". A thorough treatise of the Q-PCR and qRT-PCR techniques can be found in Bustin, S.A. (ed.) A-Z of quantitative PCR, IUL Biotechnology Series 5 (2004) 882 pages.

Today, the by far most sensitive, specific and convenient technique for quantifying microRNA is the qRT-PCR technique. Therefore in the most preferred embodiment of the resent method the expression levels of the microRNAs are determined by qRT-PCR, and a preferred example of an qRT-PCR method is the UniRT-method described in Example

4.However, similar results were obtained using the ABI Taqman qRT-PCR assay described in example 6, fig 6.

While e.g. the ratios:

$$\frac{(level\ of\ miR\ 155)}{\left(\frac{level\ of\ miR\ 203}{2} \div \frac{level\ of\ miR\ 205}{2}\right)}$$

or

$$\frac{(level\ of\ miR\ 326)}{\left(\frac{level\ of\ miR\ 203}{2} + \frac{level\ of\ miR\ 205}{2}\right)}$$

or

$$\frac{((levelofmiR155) + (levelofmiR326))/2}{((levelofmiR203) + (levelofmiR205))/2}$$

or

$$\frac{((levelofmiR155) + (levelofmiR326) + (levelofmiR663b))/3}{((levelofmiR203) + (levelofmiR205))/2}$$

all are useful estimators for S, the read-out from a typical real-time QPCR instrument is often the so-called Cp (crossing point)-value or the threshold cycle value (Ct) value both of which may be obtained by real-time qRT-PCR. Both the Ct- and the Cp-value is related to the level of e.g. a specific miR, by the relation:

$$(liniar)expressionlevelofmiRx \sim 2^{-Cp(miRx)}$$

Wherein *Cp(miRx)* designates the Cp-readout from real-time QPCR instrument specifically detecting one specific miR called miRx. Example 5 describes such an assay in details.

Accordingly, when the Cp-values are used as quantifiers of miR-levels, the expression:

$$\frac{(level\ of\ miR\ 155)}{\left(\frac{level\ of\ miR\ 203}{2} + \frac{level\ of\ miR\ 205}{2}\right)}$$

is equivalent to:

$$+Cp(miR155) - Cp(miR203)/2 - Cp(miR205)/2$$

Note that the less miR in the sample the more cycles are to be run before crossing point or the threshold cycle is reached. I.e. the larger the Cp (or Ct) value the less miR is present in the sample.

Realizing that cutaneous lymphomas of the skin and in particularly cutaneous T-cell lymphomas (CTCL) are characterized by an increased level of expression of miR-155 (and miR-326) and a decreased expression of miR-203 and miR-205 relative to the level in normal or benign skin make it possible to formulate a wide range of estimators of the clinical score S. Logistic regression is a widely used method for generating best fit linear models of data. For instance the clinical score,S, may be calculated as follows:

S = X*C(miR-155) + Y*C(miR-205) + Z*C(miR-203), wherein "C " is the threshold cycle value (Ct) or the crossing point value (Cp), and wherein X, Y, and Z are coefficients determined by linear regression, under the constraint that X + Y + Z = 0, in order to minimize the classification error.

Likevise a clinical score, S, may be calculated as: S = X*C(miR-326) + Y*C(miR-205) + Z*C(miR-203), wherein "C" is the threshold cycle value (Ct) or the crossing point value (Cp), and wherein X, Y, and Z are coefficients determined by linear regression, under the constraint that X + Y + Z = 0, in order to minimize the classification error.

Or expressed in more general terms the clinical score may be calculated as: *S = X*C(miR-155) + Y*C(miR-326) + Z*C(miR-6.63b) + W*C(miR-203) + Q*C(miR-205),* wherein "C" is the threshold cycle value (Ct) or the crossing point value (Cp), and wherein X, Y, Z, W, and Q are coefficients determined by linear regression, under the constraint that X+Y+Z+W+ Q = 0, in order to minimize the classification error.

As evidenced in Example 3 and illustrated in fig. 4 one particularly useful estimator is S = C(miR-155) - C(miR-205)/2 - C(miR-203)/2,wherein "C" is the threshold cycle value (Ct) or the crossing point value (Cp). Accordingly in the most preferred embodiment of the invention is the embodiment wherein the clinical score, S, is calculated as S = C(miR-155) - C(miR-205)/2 - C(miR-203)/2,wherein "C " is the threshold cycle value (Ct).

Using this estimator for the clinical score it is possible to formulate threshold-values, see example 3 and fig 4A.

Accordingly, in one embodiment of the invention the clinical score, S, is calculated as S = C(miR-155) - C(miR-205)/2 - C(miR-203)/2,and "C" is the crossing point value (Cp) and wherein when the clinical score "S" is lower than about 6.5, in particular lower than 6.0, the test indicates that the test skin cell sample is Cutaneous T-cell Lymphoma, and wherein when the clinical score "S" is higher than about 6.5, in particular higher than about 7.0, the test indicates that the test skin cell sample is benign.

Bearing on the same principles the a clinical score may be calculated as: *S = C(miR-326) - C(miR-205)/2 - C(miR-203)/2*wherein "C " is the threshold cycle value (Ct) or the crossing point value (Cp) or even *S = C(miR-155)/3 + C(miR-326)/3 + C(miR-663b)I3 - C(miR-205)/2 - C(miR-203)/2*wherein "C " is the threshold cycle value (Ct) or the crossing point value (Cp).

**LEGENDS**

**Figure 1.** *Expression profiles in training set for highly significant miRNAs.* We analyzed microarray measurements of 688 miRNAs in the training set of 90 samples with *t*-test to discover differences in expression between samples of subjects with CTCL and those of benign inflammatory skin diseases or healthy individuals(BDN) subjects. The 27 miRNAs that displayed highly significant (Bonferroni corrected P< 0.001) and strong differences (at least 50% change) are presented in the heat map. Samples are arranged in columns, miRNAs in rows, and both are hierarchically clustered using Euclidean distance with average linkage of nodes. Black-to white shades indicate increased relative expression; Black shades indicate reduced expression; gray indicates median expression. Top 5 most significantly induced or repressed microRNAs are shown in bold.

**Figure 2.** *Classification of CTCL and BDN.* A. Principal component analysis (PCA) plot of samples from subjects with CTCL (light gray) and those of BDN subjects (dark gray) in the training set based on the 5-microRNA profile identified by the nearest shrunken centroids (NSC) algorithm. Percentages indicate percent variance explained by that component. **B**. Classification performance in the training set using the NSC algorithm. *P*-values were calculated using Fisher's exact test. **C.** PCA plot of samples in the test set based on the 5-microRNA profile identified from the training set. **D.** Classification performance in the test set using the trained NSC algorithm.

**Figure 3.** *Classifier miRNA expressions measured by microarray and qRT-PCR.* For each microRNA, expressions are grouped according to patient type (CTCL and BDN respectively), with a small scatter on the x-axis within each group to allow better visualization of all measurements. *P*-values were calculated using *t*-test. **A.** Expressions measured by microarray. **B.** Expressions measured by qRT-PCR.

**Figure 4**. *qRT-PCR-basedclassification of samples from patients with CTCL and benign skin disease.* **A**. A Cp based sample score (*S*) were calculated for each sample. Patients are ordered by increasing values of this score. The solid line shows the cutoff between patients with CTCL (light gray) and patients with benign skin disease (dark gray). The dotted lines show the cutoffs for the low confidence region. **B**. Classification performance using the cutoffs defined in (A). *P*-values were calculated using Fisher's exact test. **C.** Receiver operator characteristic curve (ROC) showing the sensitivity and specificity for various cutoff values on the sample score of the samples. D. Relative expression of the three microRNAs used in the classification in samples from patients with CTCL and benign skin disease. Error bars indicates ±1 standard deviation. The dCp (or ΔCp) value is calculated as ΔCp = Cp, reference(=control) - Cp, observed.

**Figure. 5.** qRT-PCR-based classification of samples from patients with Mycosis Fungoides (MF) in various stages of the disease. A Cp based sample score (S) were calculated for each sample as in Fig. 4. Patients are ordered according to clinical stages (I to IV) and solid lines indicate mean sample score in each stage. Patients with a sample score below 6.52 are being classified as CTCL (c.f. Fig, 4).

**Figure 6.** The two step UniRT protocol. The principle the qRT-PCR of a microRNA serves as an example, the RNA to be analysed by the method may as well be any other small RNA molecule or even a mRNA. Step 1 is a one-tube-reaction for all microRNAs present in a sample. Step 2 is a microRNA specific qPCR using forward and reverse primer pairs for a specific microRNA. An oval indicate insertion of Locked Nucleic Acids (LNAs) in forward and reverse primers. When the method is carried out in practice the miRNAs present in a sample are firstly poly-A-tailed using a poly(A) polymerase **(SEQ ID NO: 47),** which adds adenine residues to the 3'-end of RNA molecules. Secondly, an extension primer **(SEQ ID NO: 48),** which has a poly-T-core nucleotide sequence, a 3'-end VN- or VNN-degenerate motif and a 5'-end tail, is annealed to the poly-A-tailed miRNA through hybridisation with the VN- or VNN-poly-T-sequence of the extension primer, (N=C, G, A and T; V= C, G, and A). This primer may be referred to as the Universal RT primer. Subsequently, the extension primer is extended in a reverse transcription reaction using the miRNA as template. All of these reactions are performed in a one-tube reaction. The resulting primary extension product is composed of the extension primer and the newly synthesized DNA, which is cDNA complementary to all the miRNAs in the sample. In the next step a miRNA-specific PCR is carried out. A miRNA-specific forward primer is annealed to 3'-end of the newly synthesized cDNA and the upper-strand synthesis is carried out by extending the forward primer in a DNA-polymerization reaction using the primary extension product as template. A miRNA-specific reverse primer (**SEQ ID NO: 49**) composed of a miRNA-specific 3'-end sequence, a poly-T-stretch and a 5'-end tail is then hybridized to the upper-strand and the lower-strand is synthesized by extension of the reverse primer.

**Figure 7.** *Expressions measured by qRT-PCR for the microRNAs in the classifier.* For eachmicroRNA, expressions are grouped according to patient type (BDN and CTCL respectively). Within each group, there is a small scatter on the *x*-axis to allow better visualization of all measurements.

## EXPERIMENTAL

**Microarray data preprocessing** Probe signals were background corrected by fitting a convolution of normal and exponential distributions to the foreground intensities using the background intensities as a covariate (Ritchie, 2007). Four technical replicate spots for each probe were combined to produce one signal by taking the logarithmic base-2 mean of reliable spots. If all four replicates for a given probe were judged unreliable that probe was removed from further analysis. A reference data vector *R* was calculated as the median signal of each probe across all samples. For all probe signals in a given sample, represented by the sample data vector *S*, a curve *F* was determined by locally weighted polynomial regression so as to provide the best fit between *S* and *R* (Cleveland, 1992). A normalized sample vector *M* was calculated from this by transforming it with the function *F*, so that $M = F(S)$. In this manner, all samples were normalized to the reference *R*. This normalization procedure largely follows that outlined in (Rosenfeld, 2008). Remote data points (probes in sparsely sampled intensity regions with less than 15 probes per signal unit) were considered unreliably adjusted by this method and removed before further analysis.

**Classifier statistics** Significance of differences in expression levels was assessed by a two-sided unpaired *t*-test. Class prediction was done using nearest shrunken centroid classification (Tibshirani, 2002). Briefly, a standardized centroid is computed for each class as the average expression of each microRNA in each class divided by the within-class standard deviation for that microRNA. The microRNA expression profile of a new sample is then compared to each of these class centroids, and the class, whose centroid is closest in Euclidean distance, is the predicted class for that new sample. The algorithm is trained by shrinking class centroids towards the overall centroid for all classes by a threshold amount that minimizes the misclassification error as determined through 10-fold cross validation on the training set.

### Example 1

### *miRNA expression profiling using microarrays*

Microarray analyses were used to perform miRNA profiling of 148 formalin-fixed and paraffin-embedded biopsies. Of these samples, 63 were from patients with various forms of CTCL and 85 were from patients with benign inflammatory skin diseases or healthy individuals (BDN) (Table 1).

Biopsies from the lymphoma patients were sampled during the period 1979-2004 and were collected from the archives at the Departments of Pathology at Rigshospitalet, Bispebjerg Hospital, Aalborg Sygehus and Herlev Hospital. From all lymphoma cases, tissue samples were reviewed by histology and immunohistochemistry, using as a minimum CD3, CD4, CD8, CD30, CD56, TIA-1 and Granzyme B stains. The samples were then classified in accordance with the WHO-EORTC (Olsen, 2007; Willemze, 2005; Foss, 2011; Burg, 2005) guidelines and the clinical characteristics of the cohort were reviewed to establish the final diagnoses. Biopsies from the patients with benign skin diseases and healthy controls were collected after informed consent at the Department of Dermato-Allergology, Gentofte Hospital Department of Dermatology, Bispebjerg Hospital, Department of Pathology, Rigshospitalet, and as part of clinical trials at LEO Pharma A/S and approved by the local ethical committees (H-B-2009-045 and H-1-2009-111) and the Data Protection Agency (Datatilsynet J.NR. 2010-41-4303)

Total RNA was isolated from six 10 μm tissue sections using the RecoverAll Total Nucleic Acid Isolation Kit (Applied Biosystems/Ambion, USA) according to manufacturer guidelines. Total RNA quantity and quality were checked by spectrophotometer (Nanodrop ND-1000).

From each sample 100 ng of total RNA was labeled with Hy3 fluorescent dye using the miRCURY LNA Array power labeling kit (Exiqon, Denmark). All samples were labeled the same day with the same master mix, in order to minimize technical variation. The Hy3-labelled samples were hybridized to miRCURY LNA arrays (v11.0) (Exiqon, Denmark), containing capture probes targeting all human miRNAs registered in the miRBASE version 15.0 at the Sanger Institute. The hybridization was performed overnight at 56°C according to manufacturer specifications using a Tecan HS4800 hybridization station (Tecan, Austria). Since it was not possible to hybridize all arrays in one go, samples were randomly split into 5 batches as to minimize day-to-day variation in the hybridization process. After hybridization the microarray slides were scanned using an Agilent G2565BA Microarray Scanner System (Agilent Technologies, Inc., USA) at 5μm resolution, and the resulting TIFF images were analyzed using the ImaGene 8.0 software on standard settings (BioDiscovery, Inc., USA).

Probe signals were background corrected by fitting a convolution of normal and exponential distributions to the foreground intensities using the background intensities as a covariate (Ritchie, 2007). Four technical replicate spots for each probe were combined to produce one signal by taking the logarithmic base-2 mean of reliable spots. If all four replicates for a given probe were judged unreliable that probe was removed from further analysis. A reference data vector *R* was calculated as the median signal of each probe across all samples. For all probe signals in a given sample, represented by the sample data vector *S*, a curve *F* was determined by locally weighted polynomial regression so as to provide the best fit between *S* and *R*(Clevelan d,. 1992; Rosenfeld, 2008). A normalized sample vector *M* was calculated from this by transforming it with the function *F*, so that $M= F(S)$. In this manner, all samples were normalized to the reference *R*. This normalization procedure largely follows that outlined in (Rosenfeld, 2008). Remote data points (probes in sparsely sampled intensity regions with less than 15 probes per signal unit) were considered unreliably adjusted by this method and removed before further analysis.

The samples were divided into 3/5 for training (n=90) and 2/5 for testing (n=58) with approximately equal proportion of CTCL to BDN samples in both sets. This division follows the five microarray production batches used in the study (Table 1). Out of the 688 miRNAs that passed preprocessing filtering criteria, initial statistical analysis of the training set identified 27 miRNAs showing strong (at least 50% change) and highly significant (Bonferroni corrected P-values <0.001 from t-test) differences between CTCL and benign skin diseases and normal skin (Fig 1). Thus, the expression levels of a large number of miRNAs differ considerably between patients with CTCL and patients with BDN. Essentially similar results were obtained using unsupervised hierarchical clustering based on the 209 most variable miRNAs (data not shown).

**Table 1.** Clinical characteristics of patients in the study. ndications are stratified according to age, gender, and the microarray production batch. P-values were calculated using Fisher's exact test on sums across sub-indications (the ☐ columns). MF, mycosis fungoides; SS, sezary syndrome; CALCL, cutaneous anaplastic large cell lymphoma; NOS, cutaneous T-cell lymphoma - not otherwise specified; AD, atopic dermatitis; ND, unspecified dermatosis; PP, lesional skin from psoriasis patients; PN, non-lesional skin from psoriasis patients; NN, normal skin from healthy controls.

| | Cutaneous Lymphoma (n=63) | | | | | Benign skin disease or normal skin (n=85) | | | | | | P-value |
|---|---|---|---|---|---|---|---|---|---|---|---|---|
| | MF (*n*=39) | SS (*n*=7) | CALCL (*n*=8) | NOS (*n*=9) | Σ | AD (*n* = 20) | ND (*n*=4) | PP (*n*=42) | PN (*n*=17) | NN (*n*=2) | Σ | |
| *Age (years)* * | | | | | | | | | | | | <0.001 |
| <30 | 0 | 0 | 1 | 0 | 1 | 19 | 0 | 4 | 4 | 2 | 29 | |
| 30-44 | 5 | 0 | 0 | 0 | 5 | 1 | 0 | 6 | 3 | 0 | 10 | |
| 45-59 | 9 | 1 | 1 | 2 | 13 | 0 | 1 | 19 | 7 | 0 | 27 | |
| 60-74 | 14 | 5 | 2 | 2 | 24 | 0 | 3 | 12 | 2 | 0 | 17 | |
| ≥75 | 10 | 0 | 2 | 5 | 17 | 0 | 0 | 1 | 1 | 0 | 2 | |
| *Gender** | | | | | | | | | | | | 1.00 |
| Male | 23 | 7 | 5 | 5 | 40 | 8 | 1 | 30 | 16 | 2 | 57 | |
| Female | 15 | 0 | 1 | 4 | 20 | 12 | 3 | 12 | 1 | 0 | 28 | |
| *Microarray batch* | | | | | | | | | | | | 1.00 |
| 1 | 8 | 2 | 1 | 2 | 13 | 4 | 1 | 8 | 3 | 1 | 17 | |
| 2 | 7 | 2 | 2 | 1 | 12 | 4 | 1 | 8 | 3 | 1 | 17 | |
| 3 | 7 | 1 | 2 | 2 | 12 | 4 | 1 | 8 | 3 | 0 | 16 | |
| 4 | 9 | 1 | 2 | 2 | 14 | 4 | 0 | 9 | 4 | 0 | 17 | |
| 5 | 8 | 1 | 1 | 2 | 12 | 4 | 1 | 9 | 4 | 0 | 18 | |

* Gender and age of 2 CALCL samples and one MF sample are unknown

## Example 2

***Identification of a CTCL- specific miRNA signature.***

To find a CTCL-specific signature we analyzed the training set with a nearest shrunken centroid algorithm (Tibshirani, 2002).The top 3 most induced (miR-326, miR-663b, miR-711) and 2 most repressed (miR-203, miR-205) miRNAs among the 27 highly significant miRNAs identified in the array analysis were found to be the optimal set of miRNAs for classification after shrinkage of centroids.
Significance of differences in expression levels was assessed by a two-sided unpaired *t*-test. Class prediction was done using nearest shrunken centroid classification (Tibshirani, 2002). Briefly, a standardized centroid is computed for each class as the average expression of each microRNA in each class divided by the within-class standard deviation for that microRNA. The microRNA expression profile of a new sample is then compared to each of these class centroids, and the class, whose centroid is closest in Euclidean distance, is the predicted class for that new sample. The algorithm is trained by shrinking class centroids towards the overall centroid for all classes by a threshold amount that minimizes the misclassification error as determined through 10-fold cross validation on the training set.

All five miRNAs had Bonferroni corrected P-values < $10^{-8}$ by t-test. Samples in the training set could be classified with 93% accuracy (84% sensitivity and 100% specificity, P < 0.001 by Fisher's exact test, (Fig: 2). To assess the performance of the five miRNAs in the classification of unknown samples, we used the already trained classifier on the 59 test set samples, which were classified with 97% classification accuracy (92% sensitivity and 100% specificity, P < 0.001 by Fisher's exact test) (Fig 2C and 2D). Fig. 3A shows the expression of the individual miRNAs in the classifier. For each of the five miRNAs, the normalized log2 expression values are grouped according to patient type (CTCL and BDN, respectively) (Fig. 3A).

Next, we evaluated the robustness of the classifier by ten-fold cross-validation, each time selecting different batches as training and test set (but keeping the ratio 3/5 to 2/5). The above approach identified miR-203, miR-663b, miR-205, and miR-711 in almost all cases (Table 2). One miRNA, miR-326, was only selected in 2 out of 10 divisions. Importantly, no matter which division was chosen, the classification accuracy in the test set was consistently above 90% (93.1% in average with a 99% confidence interval between 90.5% and 95.6%) (Table 2). **Table 2.** Evaluation of robustness of classification. The five microarray production batches can be divided into 3/5 training and 2/5 test sets in 10 different ways. For each division, miRNAs were selected and a classifier trained. The classifier reported in this study corresponds to the one trained in round two.

| Round | Batches selected for test set | n test (CTCL) | n test (BDN) | Batches selected for training set | n (CTCL) | n (BDN) | Accuracy (train) | Accuracy (test) | miRNAs in classifier |
|---|---|---|---|---|---|---|---|---|---|
| 1 | 1,2 | 25 | 34 | 3,4,5 | 38 | 51 | 0.94 | 0.95 | 203, 205, 299-5p, 663b, 711, 718 |
| 2 | 1,3 | 25 | 33 | 2,4,5 | 38 | 52 | 0.93 | 0.97 | 203, 205, 326, 663b, 711 |
| 3 | 1,4 | 27 | 34 | 2,3,5 | 36 | 51 | 0.95 | 0.89 | 203, 326, 663b, 718, 1252, 1249 |
| 4 | 1,5 | 25 | 35 | 2,3,4 | 38 | 50 | 0.95 | 0.92 | 203, 205, 663b, 711, 718, 1252 |
| 5 | 2,3 | 24 | 33 | 1,4,5 | 39 | 52 | 0.91 | 0.98 | 203, 205, 299-5p, 663b, 711 |

(continued)

| Round | Batches selected for test set | n test (CTCL) | n test (BDN) | Batches selected for training set | n (CTCL) | n (BDN) | Accuracy (train) | Accuracy (test) | miRNAs in classifier |
|---|---|---|---|---|---|---|---|---|---|
| 6 | 2.4 | 26 | 34 | 1,3,5 | 37 | 51 | 0.95 | 0.92 | 34b, 203, 663b, 1249, 1252 |
| 7 | 2,5 | 24 | 35 | 1,3,4 | 39 | 50 | 0.94 | 0.93 | 34b, 203, 205, 299-5p, 663b, 1249 |
| 8 | 3,4 | 26 | 33 | 1,2,5 | 37 | 52 | 0.93 | 0.94 | 203, 205, 663b, 665, 1252 |
| 9 | 3,5 | 24 | 34 | 1,2,4 | 39 | 51 | 0.93 | 0.93 | 203, 205, 663b, 711, 1285 |
| 10 | 4,5 | 26 | 35 | 1,2,3 | 37 | 50 | 0.97 | 0.89 | 203, 490-3p, 663b, 1249, 1252 |

**Example 3**

***Identification of a qR T-PCR-based miRNA classifier.***

For diagnostic purposes, qRT-PCR is more sensitive, specific, and applicable than microarrays. To confirm the microarray results above, expression levels were measured by qRT-PCR for the 5-miRNA signature on a subset of 103 samples of of the 148 samples described in example 1. The subset of samples were selected based on high RNA content as measured by NanoDrop and covered both training and test set samples.

Total RNA was isolated from six 10 μm tissue sections using the RecoverAll Total Nucleic Acid Isolation Kit (Applied Biosystems/Ambion, USA) according to manufacturer guidelines. Total RNA quantity and quality were checked by spectrophotometer (Nanodrop ND-1000). cDNA was diluted 50 x and assayed in 10 μl PCR reactions according to the protocol for miRCURY LNA™ Universal RT microRNA PCR; each microRNA was assayed once by qPCR. Negative controls excluding template from the reverse transcription reaction were performed and profiled in parallel. The amplification was performed in a LightCycler® 480 Real-Time PCR System (Roche) in 384 well plates. The amplification curves were analyzed using the Roche LC software, both for determination of Cp (by the 2nd derivative method) and for melting curve analysis. All assays were inspected for distinct melting curves and the Tm was checked to be within known specifications for the assay. Furthermore, assays must be detected with 3 Cp's less than the negative control, and with Cp<39 to be included in the data analysis. Data that did not pass these criteria were omitted from any further analysis.

MiR-103 and miR-423-5p were identified as the most stably expressed references across, samples and their average Cp, denoted Cp,ref used as normalization factor when calculating ΔCp (Vandesompele, 2002). Specifically, for the Cp measured from a given miRNA, the ΔCp (or dCp) value is calculated as ΔCp = Cp, ref- Cp, obs. The differential expression was clearly confirmed for miR-203, miR-205, and miR-326 with P-values below $10^{-11}$ and for miR-663b with a P-value below $10^{-7}$ (Fig. 3B) whereas the last miRNA, miR-711 could not be measured reliably above background fluorescence. Essentially similar results were obtained in an independent series of qRT-PCR experiments on 44 patient samples using a different qRT-PCR platform (TaqMan-data not shown). Recent studies on skin lesions from tumor stage MF and blood samples from SS patients reported on a differential expression of miR-155, miR-21, miR-24, miR-34b, miR-191, miR-486, miR-214, Let-7b, and other miRNAs (Chen, 2010; Ballabio, 2010; Holst, 2010; Narducci, 2011).

Accordingly, we performed qRT-PCR for these miRNAs and confirmed differential expression of miR-155, miR-24, miR-191, and Let-7b. In contrast, miR-34b did not achieve significance in the qRT-PCR measurements whereas miR-21 was increased in CTCL but also in a fraction of psoriasis patients (data not shown). The nearest shrunken centroid algorithm identified miR-155, miR-203, and miR-205 as the most discriminative set of miRNAs. By rewriting the equation for nearest centroid classification as an equivalent linear combination (Richard O. Duda, Peter E. Hart, David G. Stork, "Pattern Classification", Wiley Interscience, 2nd edition, 2001, pages 36-39), we obtained a simplified discriminant function, or sample score, as: S = Cp(miR-155) - Cp(miR-203)/2 - Cp(miR-205)/2.

The area under the receiver operator characteristic curve (ROC, Fig 4C) was 0.989 with 95% confidence interval between 0.9725 and 0.9996 (as calculated from 10000 stratified bootstrap replicates, Carpenter and Bithell, 2000). The significance of this result was estimated by the Wilcoxon rank-sum test to be $P < 2x10^{-16}$.

We chose thresholds by inspecting the distribution.of sample scores (Fig 4A+B) and the ROC curve (Fig 4C), and introduced a low-confidence region around the threshold (Fig. 4A+B). As miR-203 and 205 expression was decreased in CTCL (Fig. 4D) and miR-155 expression increased in CTCL (Fig 4D), the score S was smaller in CTCL when compared to benign skin disorders (Fig. 4A+B). In 103 samples, this qRT-PCR-based "minimal" miRNA classifier (miR-155, miR-203, and miR-205) distinguished patientswith CTCL from benign skin diseases with 95% classification accuracy (P<0.001, Fig. 4B) and high sensitivity/specificity as illustrated by the ROC graph in Fig. 4C (dot indicates 91% sensitivity at 97% specificity). Importantly, MF patients were classified as malignant independently of the disease stage (Fig.5). It is noteworthy that the signature differentiate even between early stages of MF and benign controls (BDN).

The sensitivity and specificity achieved by our miRNA classifier constitutes a significant improvement compared to current practice. In addition, the highly significant ROC curve, with an area under the curve (AUC) very close to 1, makes it highly plausible that these classification accuracies will extend to new samples.

Example 4

***The UniRT method***

In this example the UniRT method for amplification and quantification of small non-coding RNA molecules by use of quantitative reverse transcription polymerase chain reaction (qRT-PCR) technology is described in brief.

In brief, see fig 6 , the UniRT protocol is a two-step protocol. In STEP I the miRs present in a sample are firstly poly-A-tailed using a poly(A) polymerase, which adds adenine residues to the 3'-end of RNA molecules. Secondly, an extension primer, which has a poly-T-core nucleotide sequence, a 3'-end VN-degenerate motif and a 5'-end tail, is annealed to the poly-A-tailed miRs through hybridization with the VN-poly-T-sequence of the extension primer. Subsequently, the extension primer is extended in a reverse transcription reaction using the miR as template. The resulting primary extension product is composed of the extension primer and the newly synthesized cDNA, which complementary to the miRs in the sample.

In the next step, STEP 2, a miR-specific PCR is carried out. A miR-specific forward primer is annealed to 3'-end of the newly synthesized cDNA and the upper-strand synthesis is carried out by extending the forward primer in a DNA-polymerization reaction using the primary extension product as template. A miR-specific reverse primer composed of a miR-specific 3'-end sequence, a poly-T-stretch and a 5'-end tail is then hybridized to the upper-strand and the lower-strand is synthesized by extension of the reverse primer.

In both STEP 1 and STEP 2 the LNA's help to ensure a specific and efficient annealing of the primers to their respective targets.

**Example 5**

***Identification of microRNAs by the UniRT method***

The levels of let-7b, miR-103, miR-155, miR-184, miR-191, miR-203, miR-205, miR-24, miR-299-5p, miR-326, miR-34b, miR-423-5p, miR-663b, miR-711 and miR-718 were quantified using the UniRT qPCR method (see Example 4). In brief,

In STEP 1 of the UniRT protocol 10 ng of total RNA was used per 10 μl RT reaction having the composition:

Reaction buffer (1x Reaction buffer contains; 167 mM NaCl, 25 mM KCl, 50 mM Tris-HCl, 8 mM MoCl2, 3.33 mM DTT, 0.1 mM ATP, 0.1 mM dATP, 0.1 mM dCTP, 0.1 mM dGTP and 0.1 mM dTTP)

0.5 μM RT-primer (L2TA3: 5'-ggtactagtttttttttttttttvnn (SEQ IDNO. 1)), or (v designates cytosine, guanine and adenine residues, n designates cytosine, guanine, adenine and thymine residues).

100 unit of Moloney Murine Leukemia Virus (M-MuLV) Reverse Transcriptase (New England Biolabs, Ipswich MA)

1 unit of E.coli Poly (A) Polymerase (New England Biolabs, Ipswich MA)

The RT reactions were run in triplicate (three RT reactions per sample).

The RT reaction was incubated at 42°C for 1 hour, 95°C for 5 minutes.

Then the RT reaction was diluted 50x in water prior to qPCR analysis - STEP 2.

In STEP 2 of the UniRT protocol 1 µl of the diluted RT reaction was mixed with the PCR primer sets of Table (final concentration of each primer is 0.3 µM) for each miR and Fast start SYBR Green Master mix (Roche Diagnostics GmbH, Mannheim, Germany) according to protocol by the providers.

All qPCRs were run in singlicates (one qPCR reaction per RT reaction) in 10 µl reaction volume. The qPCR reactions were run in 384 well plates in a Roche Lightcycler 480 II (Roche Diagnostics GmbH, Mannheim, Germany).

All real-time PCR data were analyzed using the Cp(Crossing point) method calculating the relative expression ratios of the specific target miRs as the crossing point difference (ΔCp) of the specific miRs relative to one or several reference genes (Bustin, 2004; Vandesompele,2002).

The Cp-values were calculated using the Lightcycler 480 software release 1.5.0, version 1.5.0.39 accompanying the Lightcycler 480 II instrument.

**Table 3.** microRNA and Primer sequences

| | **miRBase version** | **miR sequence (5'->3')** | **F primers_Primer sequence (5'->3')** | **R primers_Primer sequence (5'->3')** |
|---|---|---|---|---|
| let-7b | 12 | tgaggtagtaggttgtgtggtt (SEQ ID NO.2) | catgaggtagtaggttg (SEQ ID NO. 17) | ggtactagttttttttttttttaaccac (SEQ ID NO. 32) |
| miR -103 | 12 | agcagcattgtacagggctatga (SEQ ID NO. 3) | agcagcattgtacagg (SEQ ID NO. 18) | gtactagttttttttttttttcatagc (SEQ ID NO. 33) |
| miR -155 | 12 | ttaatgctaatcgtgataggggt (SEQ ID NO. 4) | gacttaatgctaatcgtgat (SEQ ID NO. 19) | gtactagttttttttttttttacccta (SEQ ID NO. 34) |
| miR -184 | 12 | tggacggagaactgataagggt (SEQ ID NO. 5) | tggacggagaactgat (SEQ ID NO. 20) | gtactagttttttttttttttaccct (SEQ ID NO. 35) |
| miR -191 | 12 | caacggaatcccaaaagcagctg (SEQ ID NO. 6) | caacggaatcccaaaagc (SEQ ID NO. 21) | gtactagttttttttttttttcagc (SEQ ID NO. 36) |
| miR -203 | 12 | gtgaaatgtttaggaccactag (SEQ ID NO. 7) | gtgaaatgtttaggacca (SEQ ID NO. 22) | tgacacggaggtactagttttttttttttttctag (SEQ ID NO. 37) |
| miR -205 | 12 | tccttcattccaccggagtctg (SEQ ID NO. 8) | tccttcattccaccgga (SEQ ID NO. 23) | gtactagttttttttttttttcagact (SEQ ID NO. 38) |
| miR -24 | 12 | tggctcagttcagcaggaacag (SEQ ID NO. 9) | tggctcagttcagca (SEQ ID NO. 24) | tgacacggaggtactagttttttttttttttctgttc (SEQ ID NO. 39) |
| miR - 299-5p | 12 | tggtttaccgtcccacatacat (SEQ ID NO. 10) | tggtttaccgtcccacat (SEQ ID NO. 25) | gaggtactagttttttttttttttatgta (SEQ ID NO. 40) |
| miR -326 | 12 | cctctgggcccttcctccag (SEQ ID NO. 11) | cctctgggcccttcct (SEQ ID NO. 26) | gtactagttttttttttttttctgga (SEQ ID NO. 41) |
| miR -34b | 12 | caatcactaactccactgccat (SEQ ID NO. 12) | caatcactaactccactg (SEQ ID NO. 27) | ggtactagttttttttttttttatggc (SEQ ID NO. 42) |
| miR -423-5p | 12 | tgaggggcagagagcgagacttt (SEQ ID NO. 13) | catgggcagagagc (SEQ ID NO. 28) | aggtactagttttttttttttttaaagtc (SEQ ID NO. 43) |
| miR-663b | 12 | ggtggcccggccgtgcctgagg (SEQ ID NO. 14) | ccggccgtgcct (SEQ ID NO. 29) | gtactagttttttttttttttcctca (SEQ ID NO. 44) |
| miR -711 | 14 | gggacccagggagagacgtaag (SEQ ID NO. 15) | agggacccagggaga (SEQ ID NO. 30) | ggtactagttttttttttttttcttacg (SEQ ID NO. 45) |

(continued)

| | miRBase version | miR sequence (5'->3') | F primers_Primer sequence (5'->3') | R primers_Primer sequence (5'->3') |
|---|---|---|---|---|
| miR -718 | 14 | cttccgccccgccgggcgtcg (SEQ ID NO. 16) | tatcttccgccccgccg (SEQ ID NO. 31) | tttttttcgacgc (SEQ ID NO. 46) |
| Primer sequences are spiked with LNA (0% to 13.3% of the nucleotides) in combination with natural occurring nucleotides. Except for miR-711 and miR-718, all miRs are human miR sequences found in miR registry database release 12.0 hosted by Sanger Institute, UK (miR 12_0). miR-711 and miR-718 were not published until registry database release 14.0. | | | | |

**Example 6**

***ABI TaqMan qRT-PCR assay.***

Skin biopsy samples were obtained from patients with cutaneous T-cell lymphoma, patients with benign skin diseases including psoriasis and atopic dermatitis, and healthy subjects. FFPE blocks were sectioned in RNase free environment. Eight 10 μm tissue sections were placed in each of two 1.5 mL microcentrifuge tubes (16 sections in total) and total RNA extracted using the Recover All Total Nucleic Acid Isolation Kit (Applied Biosystems/Ambion, USA) according to manufacturer guidelines. From each sample 15 ng of total RNA was reverse transcribed to cDNA using the TaqMan Micro RNA Reverse Transcription Kit with appropriate primers (Applied Biosystems, USA) following manufacturer guidelines. Triplicates of 5 ng cDNA per reaction was each mixed with TaqMan Universal PCR Master Mix and the relevant primers and probe (Applied Biosystems, USA) and run on the Applied Biosystems ABI Prism 7900HT 384-well instrument on standard settings. The cycle threshold (Ct, the PCR cycle at which probe signal reaches a threshold value above fluorescent background) was.determined for each well.

Measurements of Ct from three technical replicate wells for each probe were combined to produce one signal by taking the mean of reliable wells. To allow identification of a stably expressed normalization factor, five commonly used small RNA references (RNU6A, RNU6B, RNU14B, RNU48 and SNORD12) were included besides the microRNAs of interest, and ranked according to their feature stability-measure across all samples (Vandesompele et al., 2002).

The two small RNAs RNU6B and SNORD12 were identified as the most stably expressed references across samples and their average Ct used as normalization factor when calculating ΔCt (Vandesompele et al., 2002). Samples were selected based on high RNA content and covered both training and test set samples. Differential expression was clearly confirmed for miR-203, miR-205, and miR-326 (Table 4).

**Table 4**. *Expressions and significance for the 5 microRNAs in the classifier.* Results are shown for both microarray and qRT-PCR measurement. The Wilcox-test-on-ranks column reports P-values from a Wilcox test performed on an un-normalized data matrix where intensities measured on each array are replaced by their rank when comparing with all intensities on that array. Significance from this test therefore indicates that the intensity for that microRNA clearly changes rank between the conditions compared (being for example among the lowest intensities on each array in one group of samples, and therefore having consistently low rank, but among the highest intensities on each array in another group of samples, and therefore having a consistently high rank on all these arrays).

| microRNA | Array | | | | | qPCR | | | |
|---|---|---|---|---|---|---|---|---|---|
| | CTCL average log2 expression | BDN average log2 expression | log2 fold change | *P* (t-test) | *P* (Wilcox test on ranks) | CTCL average expression (-ΔCt) | BDN average expression (-ΔCt) | log2 fold change (ΔΔCt) | *P* (t-test) |
| miR-203 | 6.11 | 6.88 | -0.77 | 2.50E-23 | 1.80E-20 | 5.2 | 8 | -2.8 | 4.00E-09 |
| miR-663b | 5.8 | 5.11 | 0.69 | 5.30E-17 | 3.70E-14 | 10.9 | 9.4 | 1.5 | 0.002 |
| miR-326 | 6.2 | 5.6 | 0.6 | 1.10E-15 | 8.10E-13 | 1.4 | -1 | 2.4 | 4.60E-08 |
| miR-711 | 6.13 | 5.43 | 0.7 | 2.00E-15 | 1.40E-12 | -6.3 | -4.5 | -1.8 | 0.02 |
| miR-205 | 6.25 | 7.34 | -1.1 | 5.00E-13 | 3.50E-10 | 6.2 | 9.4 | -3.2 | 3.30E-10 |

For miR-663b, the average expressions change in the same direction (increased in CTCL) for both microarray and qRT-PCR measurements, but the P-value is only 0.002 for the qRT-PCR measurements, which is a much lower significance than for miR-203, miR-205, and miR-326 (even when taking the lower number of samples tested into account). The fluorescence amplification plot for miR-663b showed a biphasic behavior with two plateaus, which indicate that the TaqMan primers and probe does not perform well. Importantly miR-711 did not achieve significance in the qRT-PCR measurements.

**BIBLIOGRAPHY**

Ambros (2003) RNA 9, 277-279

Ballabio E, Mitchell T, Kester MS van, et al. Microrna expression in sezary syndrome: identification, function, and diagnostic potential. Blood. 2010;116:1105-13.

Burg G, Kempf W, Cozzio A, et al. Who/eortc classification of cutaneous lymphomas 2005: histological and molecular aspects. Journal of cutaneous pathology. 2005;32:647-74.

Bustin SA, Nolan T. Pitfalls of quantitative real-time reverse-transcription polymerase chain reaction. Journal of biomolecular techniques. 2004; 15:155-66.

Chen J, Odenike O, Rowley JD. Leukaemogenesis: more than mutant genes. Nature reviews. Cancer. 2010;10:23-36.

Cleveland WS, Grosse E, Shyu MJ. Local regression models. Statistical Models in S. 1992;309-376:

Dereure O, Levi E, Vonderheid EC, Kadin ME. Infrequent fas mutations but no bax or p53 mutations in early mycosis fungoides: a possible mechanism for the accumulation of malignant t lymphocytes in the skin. The Journal of investigative dermatology. 2002;118:949-56.

Doom R van, Kester MS van, Dijkman R, et al. Oncogenomic analysis of mycosis fungoides reveals major differences with sezary syndrome. Blood. 2009;113:127-36.

Duda, Peter E. Hart, David G. Stork, "Pattern Classification". Wiley Interscience, 2nd edition, 2001, pages 36-39

Fits L van der, Kester MS van, Qin Y, et al. Microma-21 expression in cd4+ t cells is regulated by stat3 and is pathologically involved in sézary syndrome. The Journal of investigative dermatology. 2011;131:762-8.

Foss FM, Zinzani PL, Vose JM, et al. Peripheral t-cell lymphoma. Blood. 2011;

Garzon R, Calin G a, Croce CM. Micrornas in cancer. Annual review of medicine. 2009;60:167-79.

Girardi M, Heald PW, Wilson LD. The pathogenesis of mycosis fungoides. The New England journal of medicine. 2004;350:1978-88.

Gjerdrum LM, Woetmann a, Odum N, et al. Foxp3+ regulatory t cells in cutaneous t-cell lymphomas: association with disease stage and survival. Leukemia 2007;21:25:12-8.

Holst LM, Kaczkowski B, Gniadecki R. Reproducible pattern of microrna in normal human skin. Experimental dermatology. 2010;19:e201-5.

James Carpenter and John Bithell (2000) "Bootstrap confidence intervals: when, which, what? A practical guide for medical statisticians". Statistics in Medicine 19, 1141-1164.

Kester MS van, Ballabio E, Benner MF, et al. Mirna expression profiling of mycosis fungoides. Molecular oncology. 2011; 5, 273-80.

Krejsgaard T, Vetter-Kauczok CS, Woetmann A, et al. Ectopic expression of B-lymphoid kinase in cutaneous T-cell lymphoma. Blood. 2009;113:5896-904.

Lee BN, Duvic M, Tang CK, et al. Dysregulated synthesis of intracellular type 1 and type 2 cytokines by t cells of patients with cutaneous t-cell lymphoma. Clinical and diagnostic laboratory immunology. 1999;6:79-84.

Narducci MG, Arcelli D, Picchio MC, et al. Microrna profiling reveals that mir-21, mir486 and mir-214 are upregulated and involved in cell survival in sézary syndrome. Cell death & disease. 2011;2:e151.

Olsen E, Vonderheid E, Pimpinelli N, et al. Revisions to the staging and classification of mycosis fungoides and sezary syndrome: a proposal of the international society for cutaneous lymphomas (iscl) and the cutaneous lymphoma task force of the european organization of research and treatment of ca. Blood. 2007;110:1713-22.

Ritchie ME, Silver J, Oshlack A, et al. A comparison of background correction methods for two-colour microarrays. Bioinformatics. 2007;23:2700-7.

Rosen ST, Querfeld C. Primary cutaneous t-cell lymphomas. Hematology / the Education Program of the American Society of Hematology. American Society of Hematology. Education Program. 2006;323-30, 513.

Rosenfeld N, Aharonov R, Meiri E, et al. Micrornas accurately identify cancer tissue origin. Nature biotechnology. 2008;26:462-9.

Tibshirani RJ, Efron B. Pre-validation and inference in microarrays. Statistical applications in genetics and molecular biology. 2002; 1: Article 1.

Trautinger F, Knobler R, Willemze R, et al. Eortc consensus recommendations for the treatment of mycosis fungoides/sézary syndrome. European journal of cancer. 2006;42:1014-30.

Vandesompele J, De Preter K, Pattyn F, et al. Accurate normalization of real-time quantitative rt-pcr data by geometric averaging of multiple internal control genes. Genome biology. 2002;3:RESEARCH0034.

Willemze R, Jaffe ES, Burg G, et al. Who-eortc classification for cutaneous lymphomas. Blood. 2005;105:3768-85:

WO 2010/085966

<110> RALFKIR, Ulrik HAGEDORN, Peter AHLER, Charlotte Busch GEISLER, Carsten WOETMANN, Anders SKOV, Lone ODUM, Niels Feentved RALFKIER, Elisabeth

<120> DIAGNOSTIC MICRORNA PROFILING IN CUTANEOUS T-CELL LYMPHOMA (CTCL)

<130> 3893-0312PWO1

<150> US 61/508,838
<151> 2011-07-18

<150> US 61/508,231
<151> 2011-07-15

<160> 49

<170> PatentIn version 3.5

<210> 1
<211> 26
<212> DNA
<213> Artificial Sequence

<220>

<223> Synthetic RT-primer: L2TA3

<220>
<221> misc_feature
<222> (25) .. (26)
<223> n is a, c, g, or t

<400> 1
ggtactagtt ttttttttt tttvnn 26

<210> 2
<211> 22
<212> DNA
<213> Homo sapiens

<220>
<221> misc_feature
<222> (1)..(22)
<223> miR sequence: let-7b

<400> 2
tgaggtagta ggttgtgtgg tt 22

<210> 3
<211> 23
<212> DNA
<213> Homo sapiens

<220>
<221> misc_feature
<222> (1)..(23)
<223> miR sequence: miR-103

<400> 3
agcagcattg tacagggcta tga 23

<210> 4
<211> 23
<212> DNA
<213> Homo sapiens

<220>
<221> misc_feature
<222> (1)..(23)
<223> miR sequence: miR-155

<400> 4
ttaatgctaa tcgtgatagg ggt 23

<210> 5
<211> 22
<212> DNA
<213> Homo sapiens

<220>
<221> misc_feature
<222> (1)..(22)
<223> miR sequence: miR-184

<400> 5
tggacggaga actgataagg gt 22

<210> 6
<211> 23
<212> DNA
<213> Homo sapiens

<220>
<221> misc_feature
<222> (1)..(23)
<223> miR sequence: miR-191

<400> 6
caacggaatc ccaaaagcag ctg 23

<210> 7
<211> 22
<212> DNA
<213> Homo sapiens

<220>
<221> misc_feature
<222> (1)..(22)
<223> miR sequence: miR-203

<400> 7
gtgaaatgtt taggaccact ag 22

<210> 8
<211> 22
<212> DNA
<213> Homo sapiens

<220>
<221> misc_feature
<222> (1)..(22)
<223> miR sequence: miR-205

<400> 8
tccttcattc caccggagtc tg 22

<210> 9
<211> 22
<212> DNA
<213> Homo sapiens

<220>
<221> misc_feature
<222> (1)..(22)
<223> miR sequence: miR-24

<400> 9
tggctcagtt cagcaggaac ag 22

<210> 10
<211> 22
<212> DNA

<213> Homo sapiens

<220>
<221> misc_feature
<222> (1)..(22)
<223> miR sequence: miR-299-5p

<400> 10
tggtttaccg tcccacatac at 22

<210> 11
<211> 20
<212> DNA
<213> Homo sapiens

<220>
<221> misc_feature
<222> (1)..(20)
<223> miR sequence: miR-326

<400> 11
cctctgggcc cttcctccag 20

<210> 12
<211> 22
<212> DNA
<213> Homo sapiens

<220>
<221> misc_feature
<222> (1)..(22)
<223> miR sequence: miR-34b

<400> 12
caatcactaa ctccactgcc at 22

<210> 13
<211> 23
<212> DNA
<213> Homo sapiens

<220>
<221> misc_feature
<222> (1)..(23)
<223> miR sequence: miR-423-5p

<400> 13
tgaggggcag agagcgagac ttt 23

<210> 14
<211> 22
<212> DNA
<213> Homo sapiens

<220>
<221> misc_feature
<222> (1)..(22)
<223> miR sequence: miR-663b

<400> 14
ggtggcccgg ccgtgcctga gg 22

<210> 15
<211> 22
<212> DNA
<213> Artificial Sequence

<220>
<223> Synthetic miR sequence

<220>
<221> misc_feature
<222> (1)..(22)
<223> miR sequence: miR-711

<400> 15
gggacccagg gagagacgta ag 22

<210> 16
<211> 21
<212> DNA
<213> Artificial Sequence

<220>
<223> Synthetic miR sequence

<220>
<221> misc_feature
<222> (1)..(21)
<223> miR sequence: miR-718

<400> 16
cttccgcccc gccgggcgtc g 21

<210> 17
<211> 17
<212> DNA
<213> Artificial Sequence

<220>
<223> Synthetic F primer

<400> 17
catgaggtag taggttg 17

<210> 18
<211> 16
<212> DNA
<213> Artificial Sequence

<220>
<223> Synthetic F primer

<400> 18
agcagcattg tacagg 16

<210> 19

<211> 20
<212> DNA
<213> Artificial Sequence

<220>
<223> Synthetic F primer

<400> 19
gacttaatgc taatcgtgat 20

<210> 20
<211> 16
<212> DNA
<213> Artificial Sequence

<220>
<223> Synthetic F primer

<400> 20
tggacggaga actgat 16

<210> 21
<211> 18
<212> DNA
<213> Artificial Sequence

<220>
<223> Synthetic F primer

<400> 21
caacggaatc ccaaaagc 18

<210> 22
<211> 18
<212> DNA
<213> Artificial Sequence

<220>
<223> Synthetic F primer

<400> 22
gtgaaatgtt taggacca 18

<210> 23
<211> 17
<212> DNA
<213> Artificial Sequence

<220>
<223> Synthetic F primer

<400> 23
tccttcattc caccgga 17

<210> 24
<211> 15
<212> DNA
<213> Artificial Sequence

<220>
<223> Synthetic F primer

<400> 24
tggctcagtt cagca 15

<210> 25
<211> 18
<212> DNA
<213> Artificial Sequence

<220>
<223> Synthetic F primer

<400> 25
tggtttaccg tcccacat 18

<210> 26
<211> 16
<212> DNA
<213> Artificial Sequence

<220>
<223> Synthetic F primer

<400> 26
cctctgggcc cttcct 16

<210> 27
<211> 18
<212> DNA
<213> Artificial Sequence

<220>
<223> Synthetic F primer

<400> 27
caatcactaa ctccactg 18

<210> 28
<211> 14
<212> DNA
<213> Artificial Sequence

<220>
<223> Synthetic F primer

<400> 28
catgggcaga gage 14

<210> 29
<211> 12
<212> DNA
<213> Artificial Sequence

<220>
<223> Synthetic F primer

<400> 29
ccggccgtgc ct 12

<210> 30
<211> 15
<212> DNA
<213> Artificial Sequence

<220>
<223> Synthetic F primer

<400> 30
agggacccag ggaga 15

<210> 31
<211> 17
<212> DNA
<213> Artificial Sequence

<220>
<223> Synthetic F primer

<400> 31
tatcttccgc cccgccg 17

<210> 32
<211> 29
<212> DNA
<213> Artificial Sequence

<220>
<223> Synthetic R primer

<400> 32
ggtactagtt ttttttttt tttaaccac 29

<210> 33
<211> 28
<212> DNA
<213> Artificial Sequence

<220>
<223> Synthetic R primer

<400> 33
gtactagttt ttttttttt ttcatagc 28

<210> 34
<211> 29
<212> DNA
<213> Artificial Sequence

<220>
<223> Synthetic R primer

<400> 34
gtactagttt ttttttttt ttaccccta 29

<210> 35
<211> 27
<212> DNA
<213> Artificial Sequence

<220>
<223> Synthetic R primer

<400> 35
gtactagttt ttttttttt ttaccct 27

<210> 36
<211> 26
<212> DNA
<213> Artificial Sequence

<220>
<223> Synthetic R primer

<400> 36
gtactagttt ttttttttt ttcagc 26

<210> 37
<211> 36
<212> DNA
<213> Artificial Sequence

<220>
<223> Synthetic R primer

<400> 37
tgacacggag gtactagttt ttttttttt ttctag 36

<210> 38
<211> 28
<212> DNA
<213> Artificial Sequence

<220>
<223> Synthetic R primer

<400> 38
gtactagttt ttttttttt ttcagact 28

<210> 39
<211> 37
<212> DNA
<213> Artificial Sequence

<220>
<223> Synthetic R primer

<400> 39
tgacacggag gtactagttt ttttttttt tctgttc 37

<210> 40
<211> 30
<212> DNA

<213> Artificial Sequence

<220>
<223> Synthetic R primer

<400> 40
gaggtactag ttttttttt tttttatgta 30

<210> 41
<211> 27
<212> DNA
<213> Artificial Sequence

<220>
<223> Synthetic R primer

<400> 41
gtactagttt ttttttttt ttctgga 27

<210> 42
<211> 28
<212> DNA
<213> Artificial Sequence

<220>
<223> Synthetic R primer

<400> 42
ggtactagtt ttttttttt tttatggc 28

<210> 43
<211> 29
<212> DNA
<213> Artificial Sequence

<220>
<223> Synthetic R primer

<400> 43
aggtactagt ttttttttt tttaaagtc 29

<210> 44
<211> 27
<212> DNA
<213> Artificial Sequence

<220>
<223> Synthetic R primer

<400> 44
gtactagttt ttttttttt ttcctca 27

<210> 45
<211> 29
<212> DNA
<213> Artificial Sequence

<220>

<223> Synthetic R primer

<400> 45
ggtactagtt ttttttttt tttcttacg 29

<210> 46
<211> 14
<212> DNA
<213> Artificial Sequence

<220>
<223> Synthetic R primer

<400> 46
tttttttcg acgc 14

<210> 47
<211> 10
<212> DNA
<213> Artificial Sequence

<220>
<223> Synthetic PolyA tail of microRNA

<400> 47
aaaaaaaaaa 10

<210> 48
<211> 13
<212> DNA
<213> Artificial Sequence

<220>
<223> Synthetic extension primer

<220>
<221> misc_feature
<222> (12)..(13)
<223> n is a, c, g, or t

<400> 48
tttttttttt vnn 13

<210> 49
<211> 10
<212> DNA
<213> Artificial Sequence

<220>
<223> Synthetic DNA

<400> 49
tttttttttt 10

## Claims

**1.** A method for classifying a test skin cell sample from an individual with an inflammatory skin disease as cutaneous lymphomas comprising:

detecting microRNA expression levels at least of one of miR-155 and miR-326, and both miR-203 and miR-205,
calculating a clinical score (S) of the test cell sample based on a dataset comprising the expression levels of said microRNAs, and
classifying the test cell sample as cutaneous lymphomas or not based on the value of the clinical score.

**2.** The method of claim 1 wherein the levels of microRNA miR-155, miR-203 and miR-205 are detected and used to calculate a clinical score (S) of the test cell sample.

**3.** The method of claim 1 wherein the levels of microRNA miR-326, miR-203 and miR-205 are detected and used to calculate a clinical score (S) of the test cell sample.

**4.** The method of claim 1 wherein the levels of microRNA miR-155, miR-326, miR-203 and miR-205 are detected and used to calculate a clinical score (S) of the test cell sample.

**5.** The method of claim 1 wherein the levels of microRNA miR-155, miR-326, miR-203, miR-205 and miR-663b are detected and used to calculate a clinical score (S) of the test cell sample.

**6.** The method of any of the preceding claims, wherein the cutaneous lymphoma is Cutaneous T-cell Lymphoma (CTCL).

**7.** The method of any of the preceding claims, wherein the clinical score is calculated as a ratio of the expression level of miR-155 and the average expression levels of miR-203 and miR-205.

**8.** The method of any of claims 1 - 7, wherein the clinical score is calculated as a ratio of the expression level of miR-326 and the average expression levels of miR-203 and miR-205.

**9.** The method of any of claims 1 - 8, wherein the clinical score is calculated as a ratio of the expression level of miR-155 and miR-326 relative to the average expression levels of miR-203 and miR-205

**10.** The method of any of the preceding claims, wherein the expression levels of the microRNAs are determined by Q-PCR.

**11.** The method of claim 10, wherein the clinical score is calculated as follows:

$$S = X*C(miR\text{-}155) + Y*C(miR\text{-}205) + Z*C(miR\text{-}203)$$

wherein "C " is the threshold cycle value (Ct) or the crossing point value (Cp), and wherein X, Y, and Z are coefficients determined by linear regression, under the constraint that X + Y + Z = 0.

**12.** The method of claim 10, wherein the clinical score is calculated as follows:

$$S = X*C(miR\text{-}326) + Y*C(miR\text{-}205) + Z*C(miR\text{-}203)$$

wherein "C " is the threshold cycle value (Ct) or the crossing point value (Cp), and wherein X, Y, and Z are coefficients determined by linear regression, under the constraint that X + Y + Z = 0.

**13.** The method of claim 10, wherein the clinical score is calculated as follows:

$$S = C(miR\text{-}155) - C(miR\text{-}205)/2 - C(miR\text{-}203)/2$$

wherein "C " is the threshold cycle value (Ct) or the crossing point value (Cp).

**14.** The method of claim 10, wherein the clinical score is calculated as follows:

$$S = C(miR\text{-}326) - C(miR\text{-}205)/2 - C(miR\text{-}203)/2$$

wherein "C " is the threshold cycle value (Ct) or the crossing point value (Cp).

**15.** The method of claim 10, wherein the clinical score is calculated as follows:

$$S = C(miR\text{-}155) + C(miR\text{-}326) - C(miR\text{-}205) - C(miR\text{-}203)$$

wherein "C " is the threshold cycle value (Ct) or the crossing point value (Cp).

**16.** The method of claim 10, wherein the clinical score is calculated as follows:

$$S = C(miR\text{-}155)/3 + C(miR\text{-}326)/3 + C(miR\text{-}663b)/3 - C(miR\text{-}205)/2 - C(miR\text{-}203)/2$$

wherein "C " is the threshold cycle value (Ct) or the crossing point value (Cp).

**17.** The method of claim 13, wherein "C " is the crossing point value (Cp) and wherein when the clinical score "S" is lower than about 6.5, in particular lower than 6.0, the test indicates that the test skin cell sample is Cutaneous T-cell Lymphoma, and wherein when the clinical score "S" is higher than about 6.5, in particular higher than about 7.0, the test indicates that the test skin cell sample is benign.

**18.** The method of claim 10, wherein the clinical score is calculated as follows:

$$S = X*C(miR\text{-}155) + Y*C(miR\text{-}326) + Z*C(miR\text{-}663b) + W*C(miR\text{-}203) + Q*C(miR\text{-}205),$$

wherein "C " is the threshold cycle value (Ct) or the crossing point value (Cp), and wherein X, Y, Z, W, and Q are coefficients determined by linear regression, under the constraint that X + Y + Z + W +Q=0.

**19.** The method of any of claims 10-18, wherein the method of Q-PCR is qRT-PCR.

**Patentansprüche**

**1.** Verfahren zum Klassifizieren einer Testhautzellenprobe von einem Individuum mit einer entzündlichen Hauterkrankung als kutane Lymphome, umfassend:

Ermitteln von microRNA-Expressionshöhen bei wenigstens einem von miR-155 und miR-326 und sowohl miR-203 als auch miR-205,
Berechnen eines klinischen Score (S) der Testzellenprobe basierend auf einem die Expressionshöhen der microRNAs umfassenden Datensatz, und
Klassifizieren der Testzellenprobe als kutane Lymphome oder nicht, basierend auf dem Wert des klinischen Score.

**2.** Verfahren nach Anspruch 1, wobei die Höhen von microRNA miR-155, miR-203 und miR-205 ermittelt und verwendet werden, um den klinischen Score (S) der Testzellenprobe zu berechnen.

**3.** Verfahren nach Anspruch 1, wobei die Höhen von microRNA miR-326, miR-203 und miR-205 ermittelt und verwendet werden, um den klinischen Score (S) der Testzellenprobe zu berechnen.

**4.** Verfahren nach Anspruch 1, wobei die Höhen von microRNA miR-155, miR-326, miR-203 und miR-205 ermittelt werden und verwendet werden, um den klinischen Score (S) der Testzellenprobe zu berechnen.

5. Verfahren nach Anspruch 1, wobei die Höhen von microRNA miR-155, miR-326, miR-203, miR-205 und miR-663b ermittelt und verwendet werden, um den klinischen Score (S) der Testzellenprobe zu berechnen.

6. Verfahren nach einem der vorhergehenden Ansprüche, wobei es sich bei dem kutanen Lymphom um kutanes T-Zell-Lymphom (CTCL) handelt.

7. Verfahren nach einem der vorhergehenden Ansprüche, wobei der klinische Score als ein Verhältnis der Expressionshöhe von miR-155 und den durchschnittlichen Expressionshöhen von miR-203 und miR-205 berechnet wird.

8. Verfahren nach einem der Ansprüche 1-7, wobei der klinische Score als ein Verhältnis der Expressionshöhe von miR-326 und den durchschnittlichen Expressionshöhen von miR-203 und miR-205 berechnet wird.

9. Verfahren nach einem der Ansprüche 1-8, wobei der klinische Score als ein Verhältnis der Expressionshöhe von miR-155 und miR-326 relativ zu den durchschnittlichen Expressionshöhen von miR-203 und miR-205 berechnet wird.

10. Verfahren nach einem der vorhergehenden Ansprüche, wobei die Expressionshöhen der microRNAs durch Q-PCR bestimmt werden.

11. Verfahren nach Anspruch 10, wobei der klinische Score wie folgt berechnet wird:

$$S = X*C(miR\text{-}155) + Y*C(miR\text{-}205) + Z*C(miR\text{-}203),$$

wobei "C" der Schwellenzykluswert (Ct) oder der Kreuzungspunktwert (Cp) ist, und wobei X, Y und Z durch lineare Regression bestimmte Koeffizienten sind, unter der Bedingung, dass X + Y + Z = 0.

12. Verfahren nach Anspruch 10, wobei der klinische Score wie folgt berechnet wird:

$$S = X*C(miR\text{-}326) + Y*C(miR\text{-}205) + Z*C(miR\text{-}203),$$

wobei "C" der Schwellenzykluswert (Ct) oder der Kreuzungspunktwert (Cp) ist, und wobei X, Y und Z durch lineare Regression bestimmte Koeffizienten sind, unter der Bedingung dass X + Y + Z = 0.

13. Verfahren nach Anspruch 10, wobei der klinische Score wie folgt berechnet wird:

$$S = C(miR\text{-}155) - C(miR\text{-}205)/2 - C(miR\text{-}203)/2,$$

wobei "C" der Schwellenzykluswert (Ct) oder der Kreuzungspunktwert (Cp) ist.

14. Verfahren nach Anspruch 10, wobei der klinische Score wie folgt berechnet wird:

$$S = C(miR\text{-}326) - C(miR\text{-}205)/2 - C(miR\text{-}203)/2,$$

wobei "C" der Schwellenzykluswert (Ct) oder der Kreuzungspunktwert (Cp) ist.

15. Verfahren nach Anspruch 10, wobei der klinische Score wie folgt berechnet wird:

$$S = C(miR\text{-}155) + C(miR\text{-}326) - C(miR\text{-}205) - C(miR\text{-}203),$$

wobei "C" der Schwellenzykluswert (Ct) oder der Kreuzungspunktwert (Cp) ist.

16. Verfahren nach Anspruch 10, wobei der klinische Score wie folgt berechnet wird:

$$S = C(miR\text{-}155)/3 + C(miR\text{-}326)/3 + C(miR\text{-}663b)/3 - C(miR\text{-}205)/2 - C(miR\text{-}203)/2,$$

wobei "C" der Schwellenzykluswert (Ct) oder der Kreuzungspunktwert (Cp) ist.

**17.** Verfahren nach Anspruch 13, wobei "C" der Kreuzungspunktwert (Cp) ist und wobei der Test angibt, dass es sich bei der Hautzellenprobe um kutanes T-Zell-Lymphom handelt, wenn der klinische Score "S" niedriger ist als etwa 6,5, insbesondere niedriger als 6,0, und wobei der Test angibt, dass die Hautzellenprobe gutartig ist, wenn der klinische Score "S" höher ist als etwa 6,5, insbesondere höher als etwa 7,0.

**18.** Verfahren nach Anspruch 10, wobei der klinische Score wie folgt berechnet wird:

$$S = X*C(miR\text{-}155) + Y*C(miR\text{-}326) + Z*C(miR\text{-}663b) + W*C(miR\text{-}203) + Q*C(miR\text{-}205),$$

wobei "C" der Schwellenzykluswert (Ct) oder der Kreuzungspunktwert (Cp) ist, und wobei X, Y, Z, W und Q durch lineare Regression bestimmte Koeffizienten sind, unter der Bedingung, dass X + Y+ Z + W + Q = 0.

**19.** Verfahren nach einem der Ansprüche 10-18, wobei es sich bei dem Verfahren von Q-PCR um qRT-PCR handelt.

## Revendications

**1.** Procédé de classification d'un échantillon de cellules cutanées pour essai prélevé chez un sujet atteint d'une maladie de peau inflammatoire comme le lymphome cutané comprenant les étapes consistant à :

détecter les taux d'expression de micro-ARN d' au moins l' un de miR-155 et miR-326, et l'un et l'autre de miR-203 et miR-205,
calculer un score clinique (S) de l'échantillon de cellules pour essai basé sur un jeu de données comprenant les taux d'expression desdits micro-ARN, et
classifier l'échantillon de cellules pour essai en tant que lymphome cutané ou non en fonction de la valeur du score clinique.

**2.** Procédé selon la revendication 1, dans lequel les taux des micro-ARN miR-155, miR-203 et miR-205 sont détectés et utilisés pour calculer un score clinique (S) de l'échantillon de cellules pour essai.

**3.** Procédé selon la revendication 1, dans lequel les taux des micro-ARN miR-326, miR-203 et miR-205 sont détectés et utilisés pour calculer un score clinique (S) de l'échantillon de cellules pour essai.

**4.** Procédé selon la revendication 1, dans lequel les taux des micro-ARN miR-155, miR-326, miR-203 et miR-205 sont détectés et utilisés pour calculer un score clinique (S) de l'échantillon de cellules pour essai.

**5.** Procédé selon la revendication 1, dans lequel les taux des micro-ARN miR-155, miR-326, miR-203, miR-205 et miR-663b sont détectés et utilisés pour calculer un score clinique (S) de l'échantillon de cellules pour essai.

**6.** Procédé selon l'une quelconque des revendications précédentes, dans lequel le lymphome cutané est le lymphome cutané à cellules T (CTCL).

**7.** Procédé selon l'une quelconque des revendications précédentes, dans lequel le score clinique est calculé en tant que rapport du taux d'expression de miR-155 et des taux d'expression moyens de miR-203 et miR-205.

**8.** Procédé selon l'une quelconque des revendications 1 à 7, dans lequel le score clinique est calculé en tant que rapport du taux d'expression de miR-326 et des taux d'expression moyens de miR-203 et miR-205.

**9.** Procédé selon l'une quelconque des revendications 1 à 8, dans lequel le score clinique est calculé en tant que rapport du taux d'expression de miR-155 et miR-326 par rapport aux taux d'expression moyens de miR-203 et miR-205.

**10.** Procédé selon l'une quelconque des revendications précédentes, dans lequel les taux d'expression des micro-ARN sont déterminés par PCR quantitative (qPCR).

**11.** Procédé selon la revendication 10, dans lequel le score clinique est calculé comme suit :

$$S = X*C(miR\text{-}155) + Y*C(miR\text{-}205) + Z*C(miR\text{-}203)$$

dans lequel « C » est la valeur seuil du cycle (Ct) ou la valeur du point de coïncidence (Cp), et dans lequel X, Y et Z sont des coefficients déterminés par régression linéaire, à la condition que X + Y + Z = 0.

**12.** Procédé selon la revendication 10, dans lequel le score clinique est calculé comme suit :

$$S = X*C(miR\text{-}326) + Y*C(miR\text{-}205) + Z*C(miR\text{-}203)$$

dans lequel « C » est la valeur seuil du cycle (Ct) ou la valeur du point de coïncidence (Cp), et dans lequel X, Y et Z sont des coefficients déterminés par régression linéaire, à la condition que X + Y + Z = 0.

**13.** Procédé selon la revendication 10, dans lequel le score clinique est calculé comme suit :

$$S = C(miR\text{-}155) - C(miR\text{-}205)/2 - C(miR\text{-}203)/2$$

dans lequel « C » est la valeur seuil du cycle (Ct) ou la valeur du point de coïncidence (Cp).

**14.** Procédé selon la revendication 10, dans lequel le score clinique est calculé comme suit :

$$S = C(miR\text{-}326) - C(miR\text{-}205)/2 - C(miR\text{-}203)/2$$

dans lequel « C » est la valeur seuil du cycle (Ct) ou la valeur du point de coïncidence (Cp).

**15.** Procédé selon la revendication 10, dans lequel le score clinique est calculé comme suit :

$$S = C(miR\text{-}155) + C(miR\text{-}326) - C(miR\text{-}205) - C(miR\text{-}203)$$

dans lequel « C » est la valeur seuil du cycle (Ct) ou la valeur du point de coïncidence (Cp).

**16.** Procédé selon la revendication 10, dans lequel le score clinique est calculé comme suit :

$$S = C(miR\text{-}155)/3 + C(miR\text{-}326)/3 + C(miR\text{-}663b)/3 - C(miR\text{-}205)/2 - C(miR\text{-}203)/2$$

dans lequel « C » est la valeur seuil du cycle (Ct) ou la valeur du point de coïncidence (Cp).

**17.** Procédé selon la revendication 13, dans lequel « C » est la valeur du point de coïncidence (Cp) et dans lequel lorsque le score clinique « S » est inférieur à environ 6,5, en particulier inférieur à 6,0, le test indique que l'échantillon de cellules cutanées pour essai est le lymphome cutané à cellules T, et dans lequel lorsque le score clinique « S » est supérieur à environ 6,5, en particulier supérieur à environ 7,0, le test indique que l'échantillon de cellules cutanées pour essai est bénin.

**18.** Procédé selon la revendication 10, dans lequel le score clinique est calculé comme suit :

$$S = X*C(miR\text{-}155) + Y*C(miR\text{-}326) + Z*C(miR\text{-}663b) + W*C(miR\text{-}203) + Q*C(miR\text{-}205)$$

dans lequel « C » est la valeur seuil du cycle (Ct) ou la valeur du point de coïncidence (Cp), et dans lequel X, Y, Z, W et Q sont des coefficients déterminés par régression linéaire, à la condition que X + Y + Z +W + Q= 0.

**19.** Procédé selon l'une quelconque des revendications 10 à 18, dans lequel le procédé de qPCR est la RT-PCR quantitative (qRT-PCR).

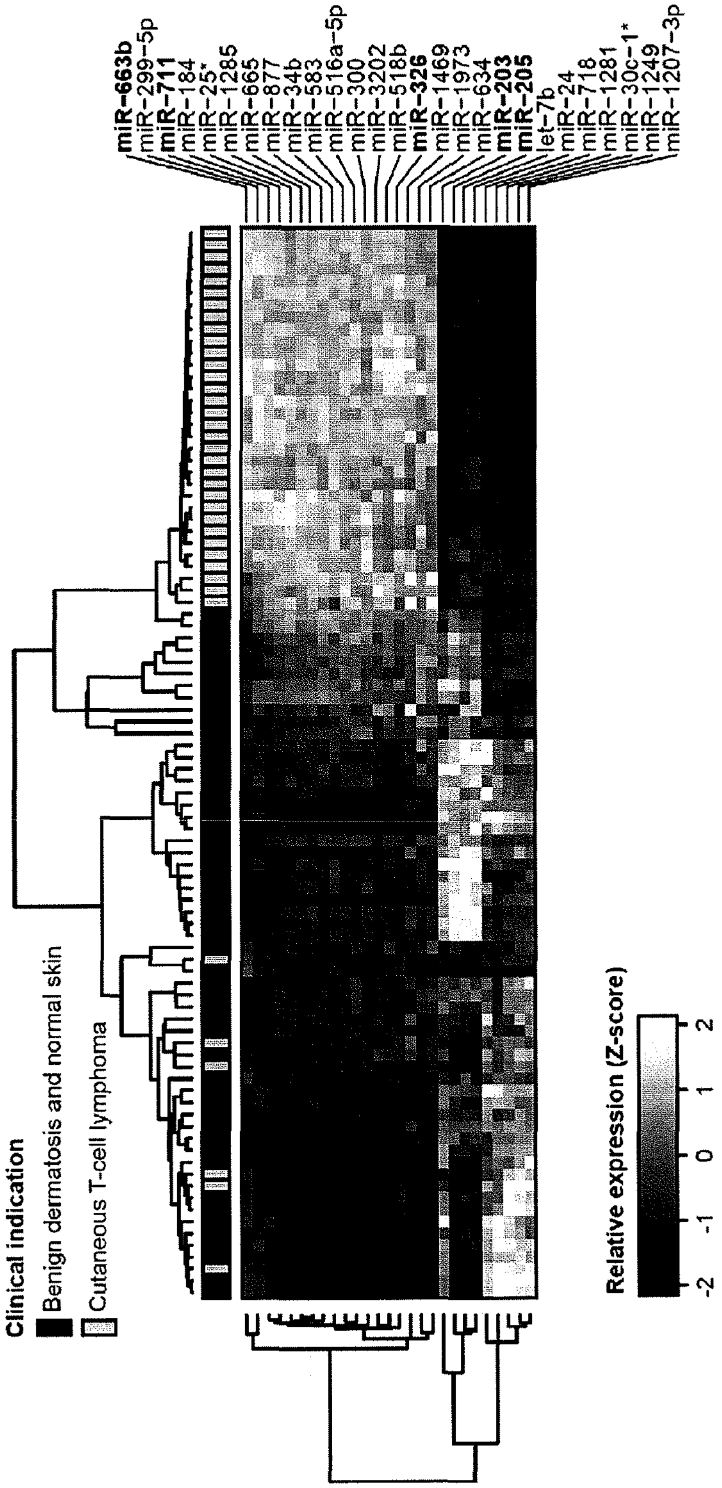
Clinical indication
Benign dermatosis and normal skin
Cutaneous T-cell lymphoma
Relative expression (Z-score)
-2
-1
0
1
2
miR-663b
miR-299-5p
miR-711
miR-184
miR-25*
miR-1285
miR-665
miR-877
miR-34b
miR-583
miR-516a-5p
miR-300
miR-3202
miR-518b
miR-326
miR-1469
miR-1973
miR-634
miR-203
miR-205
let-7b
miR-24
miR-718
miR-1281
miR-30c-1*
miR-1249
miR-1207-3p

Fig. 1

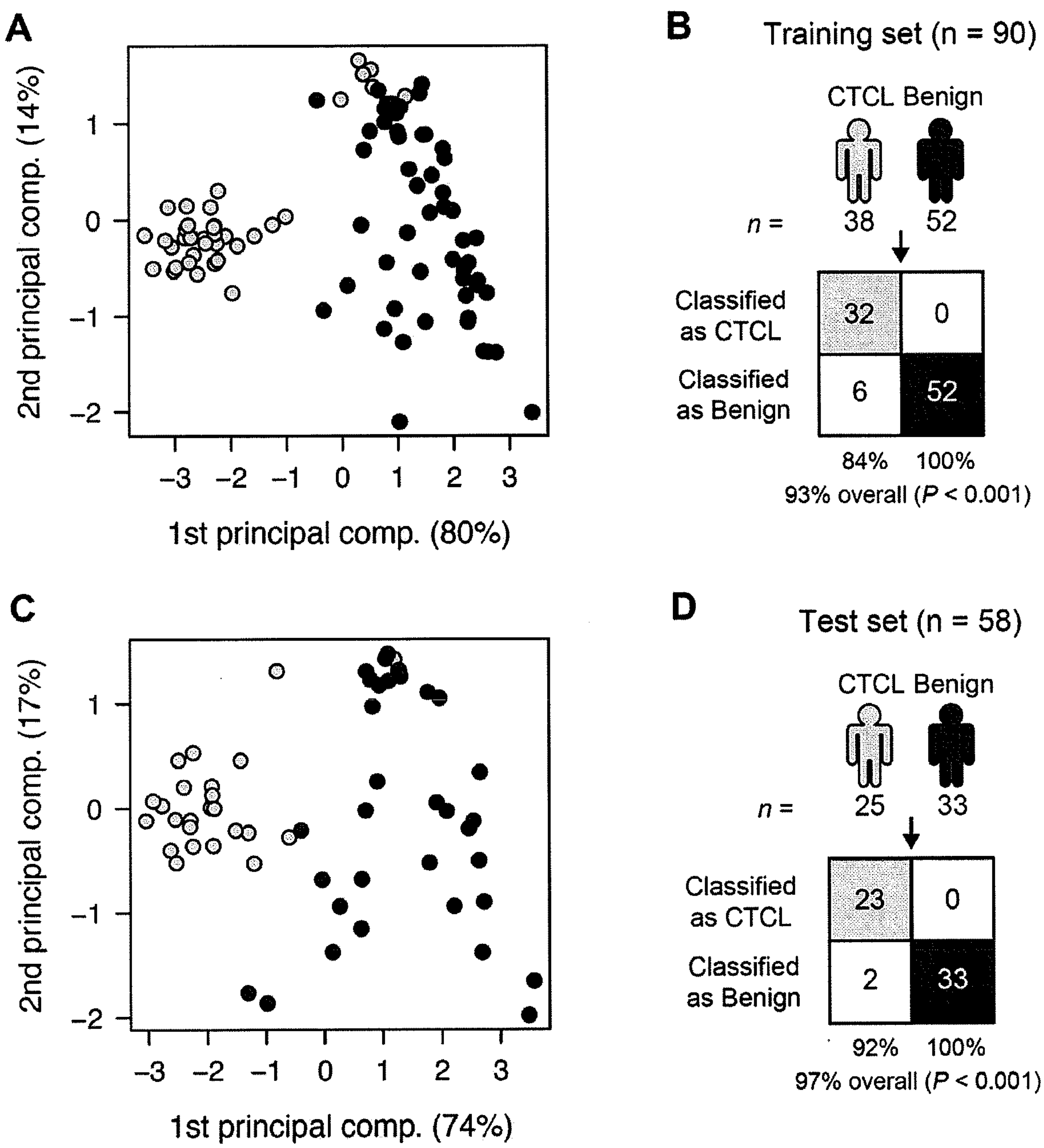
A
1
0
−1
−2
−3 −2 −1 0 1 2 3
2nd principal comp. (14%)
1st principal comp. (80%)
B
Training set (n = 90)
CTCL Benign
n =
38
52
Classified as CTCL
Classified as Benign
32
0
6
52
84%
100%
93% overall (P < 0.001)
C
1
0
−1
−2
−3 −2 −1 0 1 2 3
2nd principal comp. (17%)
1st principal comp. (74%)
D
Test set (n = 58)
CTCL Benign
n =
25
33
Classified as CTCL
Classified as Benign
23
0
2
33
92%
100%
97% overall (P < 0.001)

Fig. 2

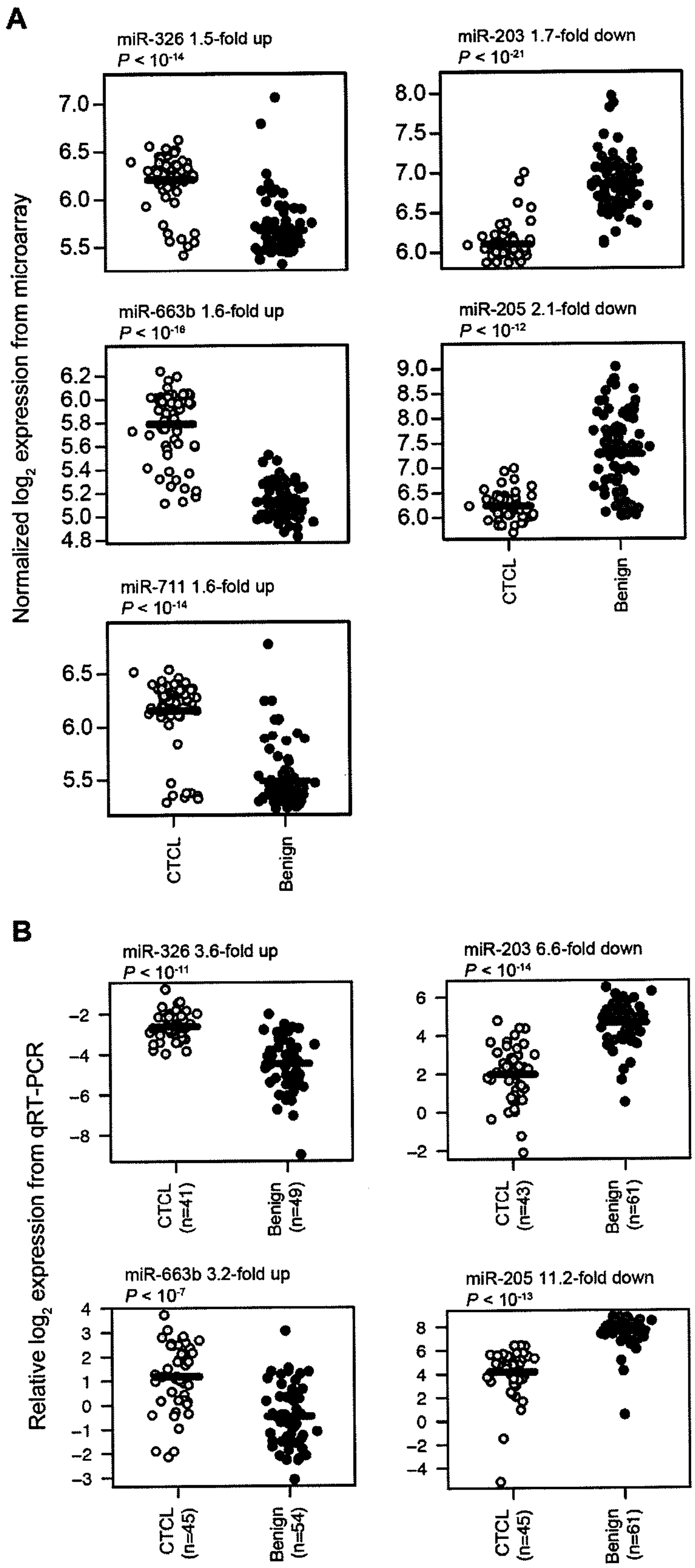
A
miR-326 1.5-fold up
P < 10-14
miR-203 1.7-fold down
P < 10-21
miR-663b 1.6-fold up
P < 10-16
miR-205 2.1-fold down
P < 10-12
miR-711 1.6-fold up
P < 10-14
Normalized log2 expression from microarray
CTCL
Benign
B
miR-326 3.6-fold up
P < 10-11
miR-203 6.6-fold down
P < 10-14
miR-663b 3.2-fold up
P < 10-7
miR-205 11.2-fold down
P < 10-13
Relative log2 expression from qRT-PCR
CTCL (n=41)
Benign (n=49)
CTCL (n=43)
Benign (n=61)
CTCL (n=45)
Benign (n=54)
CTCL (n=45)
Benign (n=61)

Fig. 3

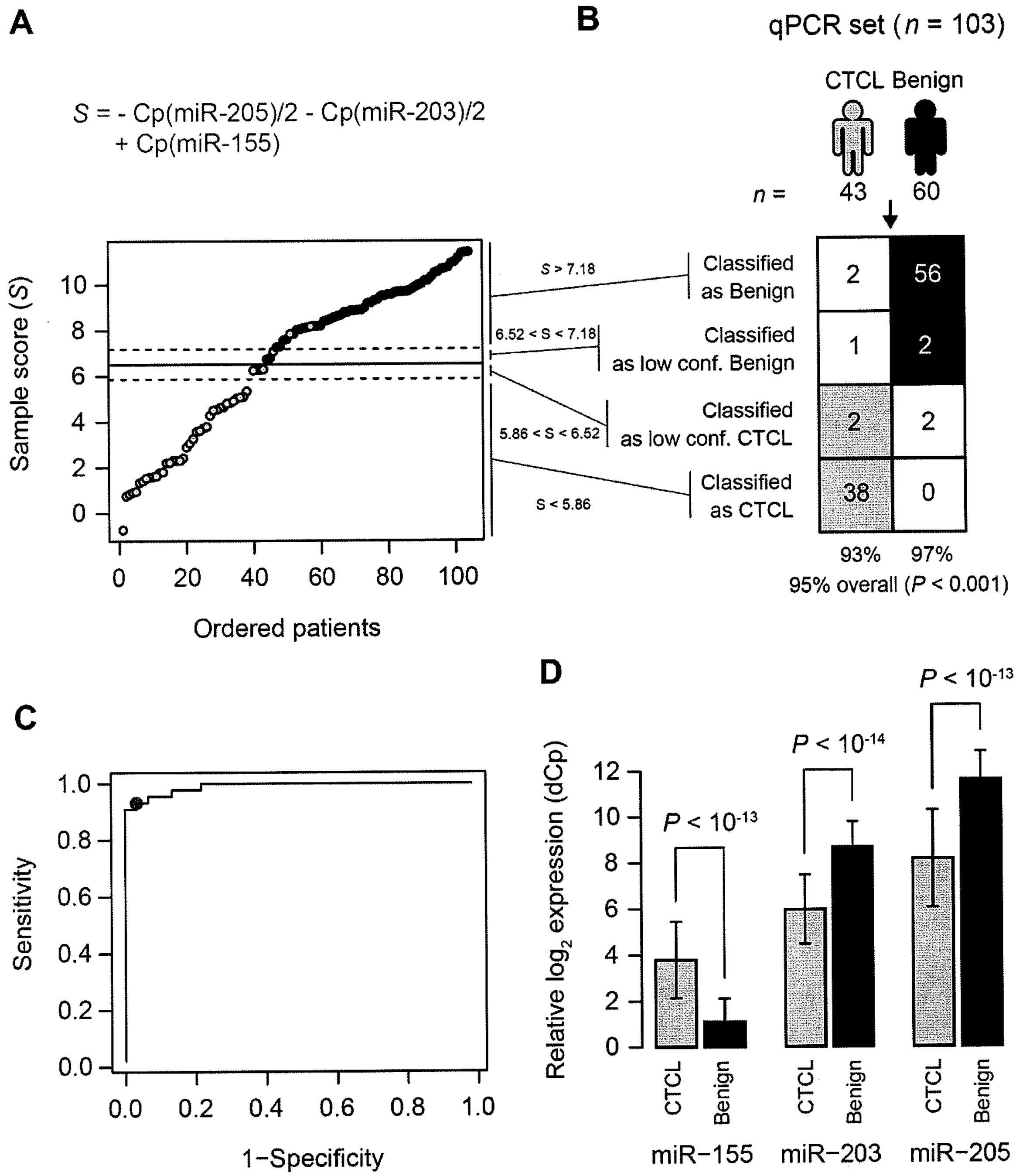
A
S = - Cp(miR-205)/2 - Cp(miR-203)/2
+ Cp(miR-155)
Sample score (S)
Ordered patients
S > 7.18
6.52 < S < 7.18
5.86 < S < 6.52
S < 5.86
B
qPCR set (n = 103)
CTCL Benign
n = 43 60
Classified as Benign
Classified as low conf. Benign
Classified as low conf. CTCL
Classified as CTCL
2 56
1 2
2 2
38 0
93% 97%
95% overall (P < 0.001)
C
Sensitivity
1−Specificity
D
Relative log2 expression (dCp)
P < 10-13
P < 10-14
P < 10-13
CTCL Benign
miR−155 miR−203 miR−205

Fig. 4

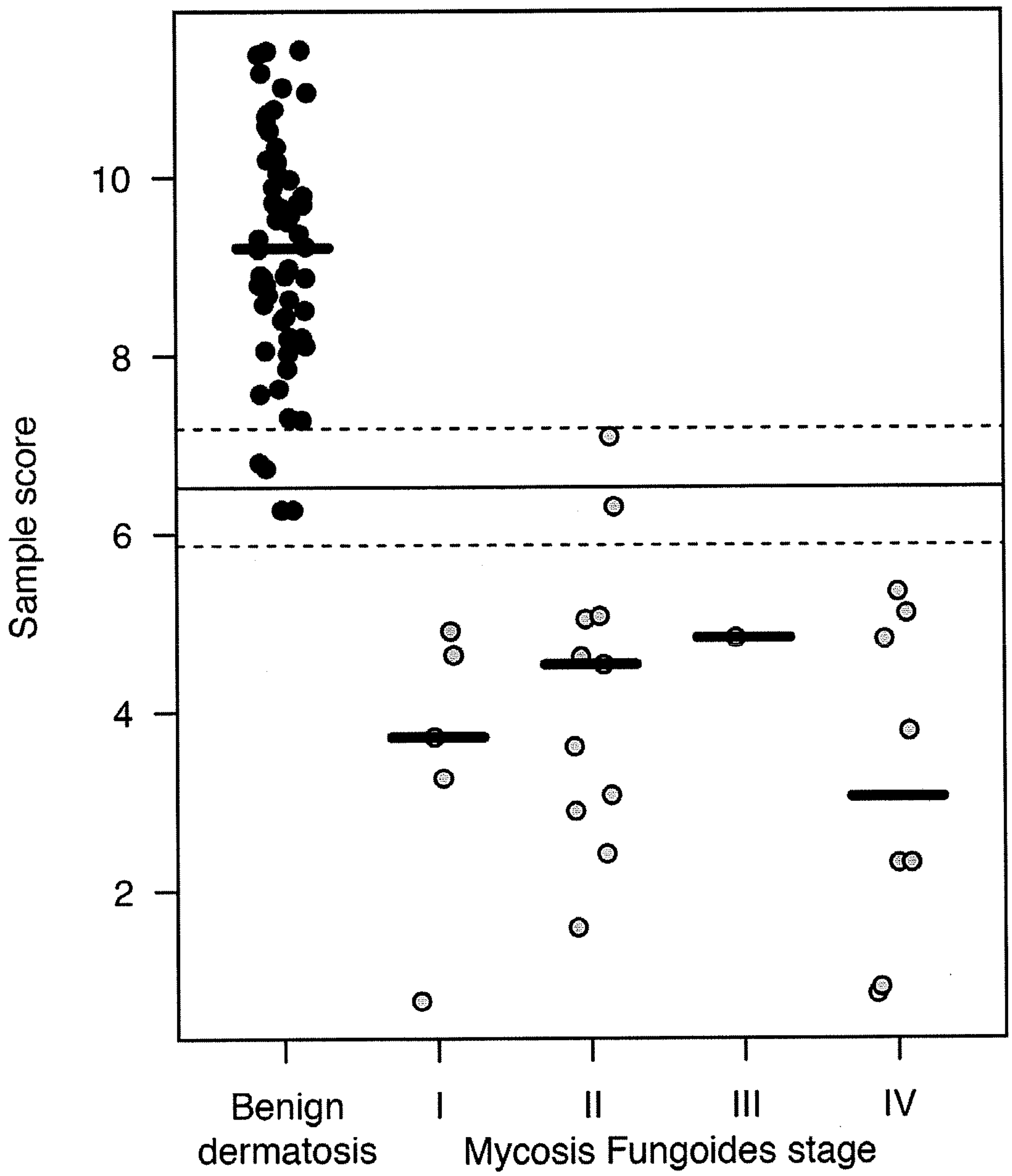
10
8
6
4
2
Sample score
Benign
dermatosis
I
II
III
IV
Mycosis Fungoides stage

Fig. 5

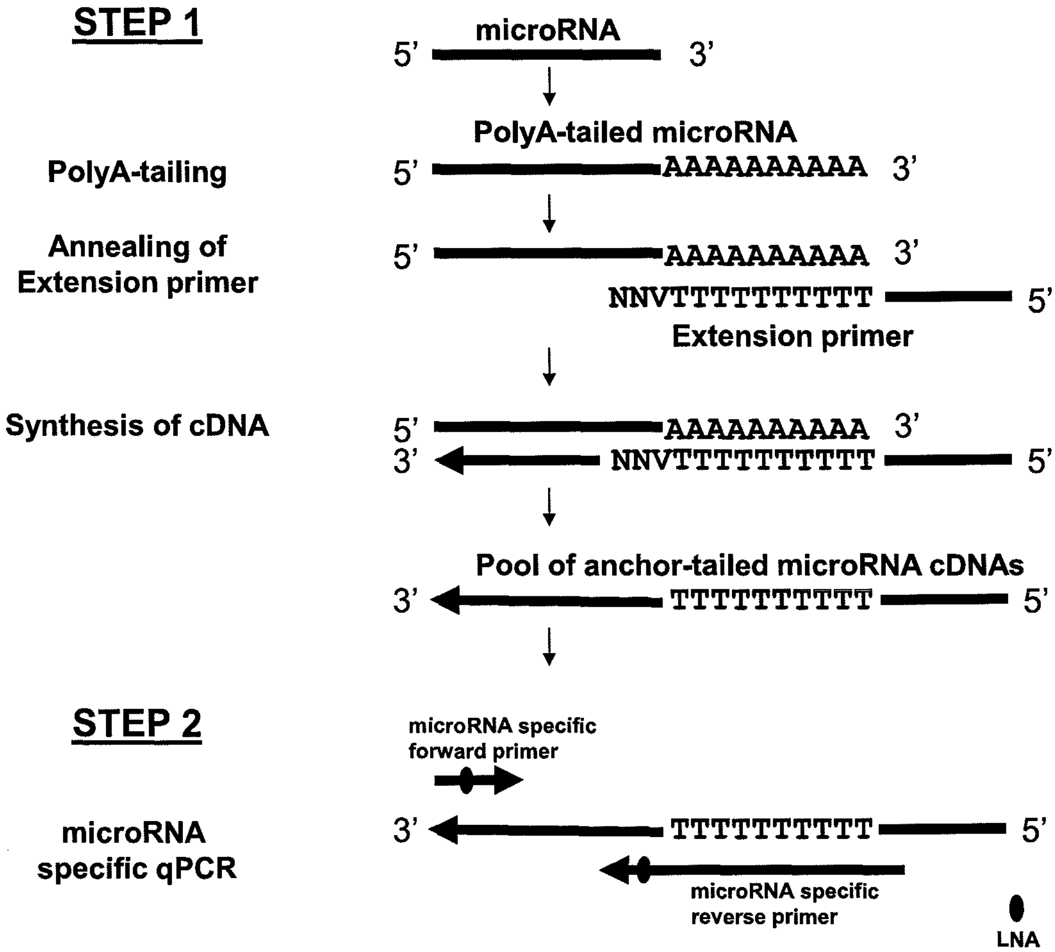
STEP 1
microRNA
5'
3'
PolyA-tailed microRNA
PolyA-tailing
5'
AAAAAAAAAA
3'
Annealing of Extension primer
5'
AAAAAAAAAA
3'
NNVTTTTTTTTTTT
5'
Extension primer
Synthesis of cDNA
5'
AAAAAAAAAA
3'
3'
NNVTTTTTTTTTTT
5'
Pool of anchor-tailed microRNA cDNAs
3'
TTTTTTTTTT
5'
STEP 2
microRNA specific forward primer
microRNA specific qPCR
3'
TTTTTTTTTT
5'
microRNA specific reverse primer
LNA

Fig. 6

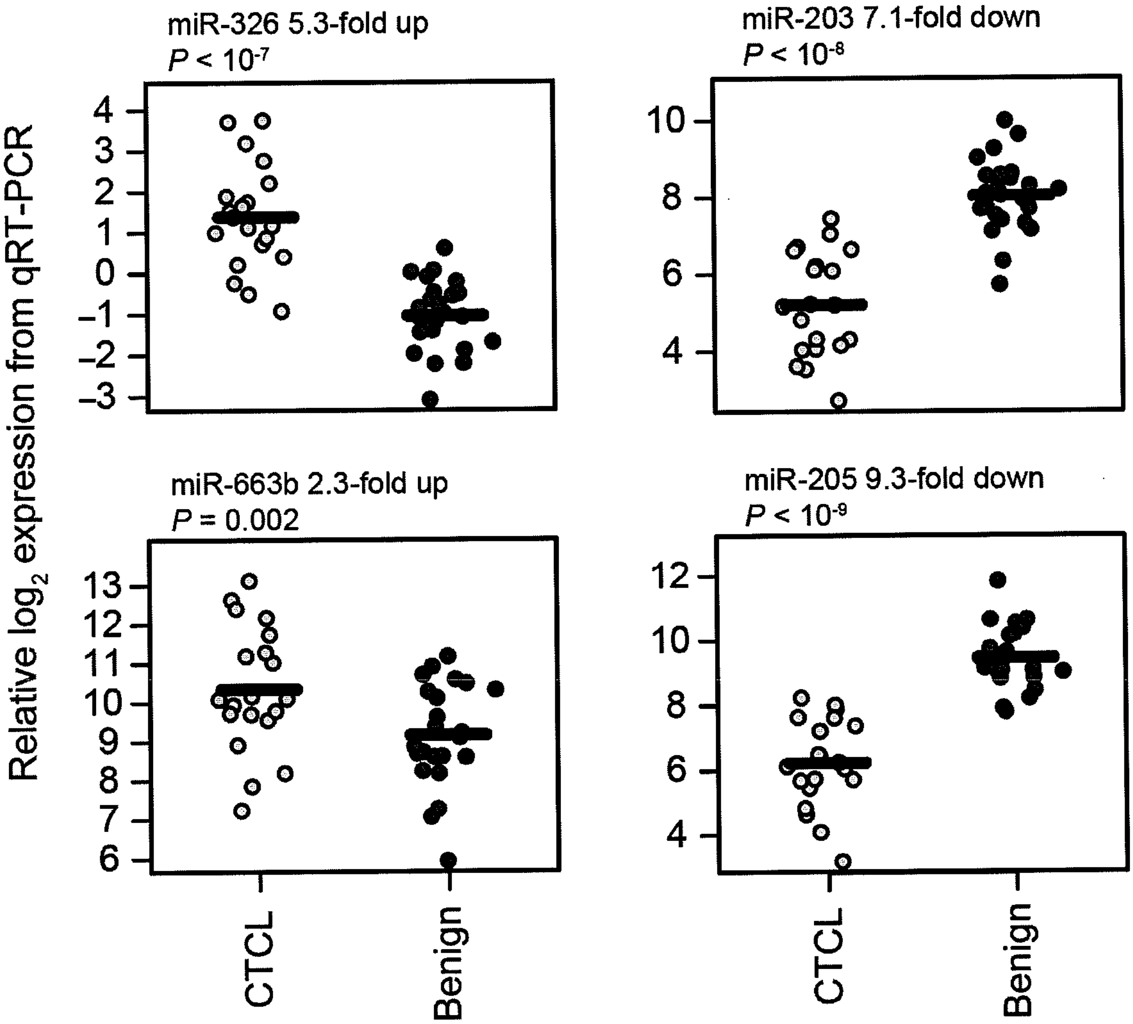
Relative $\log_2$ expression from qRT-PCR
miR-326 5.3-fold up
P < 10^-7
4
3
2
1
0
−1
−2
−3
miR-203 7.1-fold down
P < 10^-8
10
8
6
4
miR-663b 2.3-fold up
P = 0.002
13
12
11
10
9
8
7
6
miR-205 9.3-fold down
P < 10^-9
12
10
8
6
4
CTCL
Benign
CTCL
Benign

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010085966 A
- US 61508838 B
- US 61508231 B


### Non-patent literature cited in the description


- **AMBROS ; 2003 et al.** *RNA,* vol. 9, 277-279
- A-Z of quantitative PCR. IUL Biotechnology Series 5. 2004, 882
- **RICHARD O. DUDA ; PETER E. HART ; DAVID G. STORK.** Pattern Classification. Wiley Interscience, 2001, 36-39
- **AMBROS.** *RNA,* 2003, vol. 9, 277-279
- **BALLABIO E ; MITCHELL T ; KESTER MS VAN et al.** Microrna expression in sezary syndrome: identification, function, and diagnostic potential. *Blood,* 2010, vol. 116, 1105-13
- **BURG G ; KEMPF W ; COZZIO A et al.** Who/eortc classification of cutaneous lymphomas 2005: histological and molecular aspects. *Journal of cutaneous pathology,* 2005, vol. 32, 647-74
- **BUSTIN SA ; NOLAN T.** Pitfalls of quantitative real-time reverse-transcription polymerase chain reaction. *Journal of biomolecular techniques,* 2004, vol. 15, 155-66
- **CHEN J ; ODENIKE O ; ROWLEY JD.** Leukaemogenesis: more than mutant genes. *Nature reviews. Cancer,* 2010, vol. 10, 23-36
- **CLEVELAND WS ; GROSSE E ; SHYU MJ.** Local regression models. *Statistical Models in S.,* 1992, 309-376
- **DEREURE O ; LEVI E ; VONDERHEID EC ; KADIN ME.** Infrequent fas mutations but no bax or p53 mutations in early mycosis fungoides: a possible mechanism for the accumulation of malignant t lymphocytes in the skin. *The Journal of investigative dermatology,* 2002, vol. 118, 949-56
- **DOOM R VAN ; KESTER MS VAN ; DIJKMAN R et al.** Oncogenomic analysis of mycosis fungoides reveals major differences with sezary syndrome. *Blood,* 2009, vol. 113, 127-36
- **DUDA ; PETER E. HART ; DAVID G. STORK.** Pattern Classification. Wiley Interscience, 2001, 36-39
- **FITS L VAN DER ; KESTER MS VAN ; QIN Y et al.** Microma-21 expression in cd4+ t cells is regulated by stat3 and is pathologically involved in sézary syndrome. *The Journal of investigative dermatology,* 2011, vol. 131, 762-8
- **FOSS FM ; ZINZANI PL ; VOSE JM et al.** Peripheral t-cell lymphoma. *Blood,* 2011
- **GARZON R ; CALIN G A ; CROCE CM.** Micrornas in cancer. *Annual review of medicine,* 2009, vol. 60, 167-79
- **GIRARDI M ; HEALD PW ; WILSON LD.** The pathogenesis of mycosis fungoides. *The New England journal of medicine,* 2004, vol. 350, 1978-88
- **GJERDRUM LM ; WOETMANN A ; ODUM N et al.** Foxp3+ regulatory t cells in cutaneous t-cell lymphomas: association with disease stage and survival. *Leukemia,* 2007, vol. 21 (25), 12-8
- **HOLST LM ; KACZKOWSKI B ; GNIADECKI R.** Reproducible pattern of microrna in normal human skin. *Experimental dermatology,* 2010, vol. 19, e201-5
- **JAMES CARPENTER ; JOHN BITHELL.** Bootstrap confidence intervals: when, which, what? A practical guide for medical statisticians. *Statistics in Medicine,* 2000, vol. 19, 1141-1164
- **KESTER MS VAN ; BALLABIO E ; BENNER MF et al.** Mirna expression profiling of mycosis fungoides. *Molecular oncology,* 2011, vol. 5, 273-80
- **KREJSGAARD T ; VETTER-KAUCZOK CS ; WOETMANN A et al.** Ectopic expression of B-lymphoid kinase in cutaneous T-cell lymphoma. *Blood,* 2009, vol. 113, 5896-904
- **LEE BN ; DUVIC M ; TANG CK et al.** Dysregulated synthesis of intracellular type 1 and type 2 cytokines by t cells of patients with cutaneous t-cell lymphoma. *Clinical and diagnostic laboratory immunology,* 1999, vol. 6, 79-84
- **NARDUCCI MG ; ARCELLI D ; PICCHIO MC et al.** Microrna profiling reveals that mir-21, mir486 and mir-214 are upregulated and involved in cell survival in sézary syndrome. *Cell death & disease,* 2011, vol. 2, e151

- **OLSEN E ; VONDERHEID E ; PIMPINELLI N et al.** Revisions to the staging and classification of mycosis fungoides and sezary syndrome: a proposal of the international society for cutaneous lymphomas (iscl) and the cutaneous lymphoma task force of the european organization of research and treatment of ca. *Blood,* 2007, vol. 110, 1713-22
- **RITCHIE ME ; SILVER J ; OSHLACK A et al.** A comparison of background correction methods for two-colour microarrays. *Bioinformatics,* 2007, vol. 23, 2700-7
- Primary cutaneous t-cell lymphomas. **ROSEN ST ; QUERFELD C.** Hematology / the Education Program of the American Society of Hematology. American Society of Hematology, 2006, 323-30, 513
- **ROSENFELD N ; AHARONOV R ; MEIRI E et al.** Micrornas accurately identify cancer tissue origin. *Nature biotechnology,* 2008, vol. 26, 462-9
- **TIBSHIRANI RJ ; EFRON B.** Pre-validation and inference in microarrays. *Statistical applications in genetics and molecular biology,* 2002, vol. 1
- **TRAUTINGER F ; KNOBLER R ; WILLEMZE R et al.** Eortc consensus recommendations for the treatment of mycosis fungoides/sézary syndrome. *European journal of cancer,* 2006, vol. 42, 1014-30
- **VANDESOMPELE J ; DE PRETER K ; PATTYN F et al.** Accurate normalization of real-time quantitative rt-pcr data by geometric averaging of multiple internal control genes. *Genome biology,* 2002, vol. 3
- **WILLEMZE R ; JAFFE ES ; BURG G et al.** Who-eortc classification for cutaneous lymphomas. *Blood,* 2005, vol. 105, 3768-85